# EUROPEAN PATENT APPLICATION

(11) **EP 1 760 092 A1**
(43) Date of publication of application: **07.03.2007**
(21) Application number: 05018634.5
(22) Date of filing: 26.08.2005
(51) Int. Cl.: C07K 19/00, C12N 15/62, C12N 5/10, C12N 15/63, G01N 33/50

(54) **System for screening cells for high expression of a protein of interest**

(71) Applicant: Applied Research Systems ARS Holding N.V., Curacao (AN)
(72) Inventor: Dupraz, Philippe, 1023 Crissier (CH); Kobr, Michel, 1026 Echandens (CH)
(74) Representative: Graf von Stosch, Andreas

(57) **Abstract**

This invention refers to industrial production of proteins. More particularly, the invention refers to a fusion protein as a novel chimeric selection marker comprising a peptide conferring resistance to an antibiotic, or a fragment, allelic variant, splice variant or mutein thereof, and at least one sequence comprising SEQ ID NO: 1, 2 or 3, preferably for producing a protein of interest (POI). The inventive chimeric selection marker exhibits: (i) a resistance to an antibiotic; and (ii) a fluorescence activity upon binding of a ligand to the sequence comprising SEQ ID NO: 1, 2 or 3. The invention further refers to nucleic acids encoding the inventive fusion protein and to expression vectors, comprising the inventive fusion protein and additionally the protein of interest (POI). Finally, uses of the inventive chimeric selection marker for screening cells for high expression of a protein of interest (POI) are disclosed.

## Description

This invention refers to industrial production of proteins. More particularly, the invention refers to a fusion protein as a novel chimeric selection marker comprising a peptide conferring resistance to an antibiotic, or a fragment, allelic variant, splice variant or mutein thereof, and at least one sequence comprising SEQ ID NO: 1, 2 or 3, preferably for producing a protein of interest (POI). The inventive chimeric selection marker exhibits: (i) a resistance to an antibiotic; and (ii) a fluorescence activity upon binding of a ligand to the sequence comprising SEQ ID NO: 1, 2 or 3. The invention further refers to nucleic acids encoding the inventive fusion protein and to expression vectors, comprising the inventive fusion protein and additionally the protein of interest (POI). Finally, uses of the inventive chimeric selection marker for screening cells for high expression of a protein of interest (POI) are disclosed.

Transfection of DNA into mammalian cells is a common technique, often used to study the effects of transient protein expression or to develop stable cell lines. Such methods allow to study the structure-function relationship of proteins of interest (POI). However, it is difficult to monitor the success of these experiments until the endpoint of reaction is reached. Particularly in the case of transient expression, it is desirable to determine e.g. the transfection efficiency or the expression rate. However, reporter molecules used for the control of the transfection efficiency or the expression rate, e.g. chloramphenicol acetyltransferase or -galactosidase, typically require cells to be fixed and incubated with an exogeneous substrate, e.g. an heterologous gene. Introducing heterologous genes into animal host cells and screening for expression of the added genes is a lengthy and complicated process. Some major problems to be overcome are e.g.: (i) the construction of large expression vectors; (ii) the transfection and selection of clones with stable long-term expression, eventually in the absence of selective pressure; and (iii) screening for high expression rates of the heterologous protein of interest.

Selection of the clones, having integrated the gene of interest and/or highly expressing the protein of interest, is typically performed using one marker system which allows a skilled person to pre-select clones by means of a simple selection system.

One typical approach is the use of selection markers conferring resistance to selective pressure. Most of these selection markers confer resistance to an antibiotic such as, e.g. neomycin, kanamycin, hygromycin, gentamycin, chloramphenicol, puromycin, zeocin or bleomycin. When generating cell clones expressing a gene of interest from expression vectors, host cells are typically transfected with a plasmid DNA vector encoding both a protein of interest and selection marker as mentioned above on the same vector. However, the plasmid capacity to incorporate gene sequences is normally limited and, accordingly, the selection marker has to be expressed by a second plasmid, which is co-transfected with the plasmid comprising the gene of interest.

Stable transfection typically leads to random integration of the expression vector into the genome of the host cell. Use of selective pressure, e.g. by administering an antibiotic to the medium, eliminates all cells that did not integrate the vector containing the selection marker providing resistance to the respective antibiotic or selective pressure in general. If the selection marker is located on the same vector as the gene of interest, or alternatively, if the selection marker is located on a second vector being co-transfected with the vector comprising the gene of interest, the cells will express both the selection marker and the gene of interest. It is frequently observed, however, that the expression level of the gene of interest is highly variable depending on the integration site.

Furthermore, when removing selective pressure from the system, it is frequently observed that expression becomes unstable or even vanishes. Only a small number of initial transfectants are thus providing high and stable long-term expression and it is extremely tedious to identify these clones in large candidate populations. Thus, it would be advantageous in these systems to cultivate candidate clones in the absence of selective pressure in a first step, following an initial period of selection for stable transfection, in order to obtain a large candidate population. Subsequently, in a second step, candidate clones may be screened for expression of a gene of interest. However, then no selection can be carried out upon applying selective pressure as known for prior art methods.

In another approach, screening for clones highly-expressing the protein of interest can be carried out by methods directly revealing the presence of high protein amounts. Typically, immunologic methods, such as ELISA or immunohistochemical staining, are applied to detect the integrated product either intracellularly or in cell culture supernatants. These methods are often tedious, expensive, time-consuming, and typically not amenable to High-Throughput-Screening (HTS)-Assays. It is to be noted that, in addition, an antibody specific for the expressed protein must be available in order to enable detection of the expressed protein.

Attempts to quantify the protein amounts by Fluorescence-Activated Cell Sorting (FACS) have also been made, but only with a limited success, especially in the case of secreted proteins (see e.g. Borth et al. (2000); Biotechnol. Bioeng. 71, 266-273). The FACS technology is based on the step of tagging subpopulations of cells with a detectable marker and sorting preferred cells by means of a signal excited by this marker.

Numerous easily detectable markers are available in the art. They usually correspond to enzymes which act on chromogenic or luminogenic substrates such as, e.g. β-glucuronidase, chloramphenicol acetyltransferase, nopaline synthase, β-galactosidase, luciferase and secreted alkaline phosphatase (SEAP). Fluorescent proteins such as, e.g. Green Fluorescent Protein (GFP), or alternatively the synthetic peptide as described by Griffin et al. ("Specific covalent labeling of recombinant protein molecules inside live cells, Science, 1998, Jul 10; 281 (5374): 269-72) may be used as a detectable marker in FACS. The activity of all these proteins and peptides can be measured by standard assays that may be established in High-Throughput-Screening (HTS)-formats.

In a different approach, selection of clones, having integrated the gene of interest and/or highly expressing the protein of interest, can be performed by using more than one of the marker systems as disclosed above.

One general approach for the screening of high expression rates of the protein of interest refers to the use of two detectable selection markers, each having selection properties. Such a selection marker system, having two separate markers, makes use of a detectable marker and an additional marker, expressed from the same vector as the gene of interest (see e.g. Chesnut et al. (1996); J. Immunol. Methods 793, 17-27). The underlying idea of this concept of using such a detectable selection marker system is to establish a correlation between the expression of the gene of interest and the additional marker due to co-expression of the two separate genes on the same vector.

The drawback of this approach is the use of yet another expression cassette for the additional selection marker. This renders the expression vector rather bulky by hosting expression units comprising a promoter, a cDNA and polyadenylation signals for at least three proteins (i.e., the gene of interest, the selection marker and an additional marker). For multi-chain proteins the situation becomes even more complex. Alternatively, individual plasmid vectors expressing the three genes, which encode (a) the protein of interest, (b) the selection marker and (c) the additional selection marker, respectively, could be co-transfected. However, it is likely that the vectors will be either integrated at different loci, or exhibit varying and uncorrelated and additionally very low expression rates. Moreover, proteins expressed with very low expression rates may be inactive or misfolded due to ineffective or defective translation. As a consequence, in such constructs, the protein of interest should not exceed a defined molecular weight (which, however, depends on the expression system used) when using bulky detectable markers in order to allow effective translation to at least some extent. Nevertheless, this significantly lowers applicability of the above method.

Another approach to overcome the above limitations consists in the use of a chimeric marker that combines the functional properties of a selection marker and of a detectable marker. Such an approach was described, e.g. by Bennett et al. (1998, Biotechniques 24, 478-482). Bennett et al. (1998) disclose the GFP-Zeo^{R} marker, which confers resistance to the Zeocin antibiotic, the expression of which can be monitored by fluorescence microscopy.

Finally, the use of a fusion protein comprising a luciferase and the puromycin N-acetyl transferase was suggested in the art, particularly the use of luciferases derived from a firefly such as, e.g., photinus pyralis, Luciola cruciata, Luciola lateralis or Photuris pennsylvanica, from Renilla reniformis (sea pansy) or from Vargula hilgendorfii (sea firefly) fused in frame with puromycin N-acetyl transferase. This fusion protein allows to combine the functional properties of a selection marker (puromycin) and a detectable marker (luciferase activity). However, such chimeric markers evoke similar problems as the systems above. E.g. incorporation of bulky detectable markers such as luciferases or GFP into cells are typically based on rather bulky expression cassettes, which - as explained above - may lead to low expression rates. Thus, the problems resulting from the use of state-of-the-art markers are not yet solved. There still exists a need of providing efficient chimeric markers. The provision of a novel, alternative and powerful chimeric marker would be extremely useful in the field of industrial production of therapeutic proteins and for screening for high-expressing clones.

Therefore, the object underlying the present invention is to provide a chimeric marker system allowing both to select cells and to monitor expression of a protein of interest (POI), without being limited by a strict size limitation for the proteins of interest.

The above object is solved by an inventive chimeric selection marker provided as a fusion protein comprising a peptide conferring resistance to an antibiotic, or a fragment, allelic variant, splice variant or mutein thereof and at least one sequence comprising SEQ ID NO: 1, 2 or 3, wherein the inventive chimeric selection marker exhibits: (i) a resistance to said antibiotic; and (ii) a fluorescence activity upon binding of a ligand to said sequence comprising SEQ ID NO: 1, 2 or 3. If the inventive chimeric selection marker is incorporated into the cell, the cell is characterized by cell survival upon addition of the corresponding antibiotic and emits fluorescent light, if a suitable ligand is added.

The inventive fusion protein comprises as a first component a peptide conferring resistance to an antibiotic. This antibiotic is preferably selected from neomycin, kanamycin, neomycin-kanamycin, hygromycin, gentamycin, chloramphenicol, puromycin, zeocin or bleomycin, respectively.

The peptides used as a first component and conferring resistance to these antibiotics are preferably encoded by a corresponding resistance gene. Preferably, the resistance gene is selected from the resistance genes for the above mentioned antibiotics, e.g. the gene encoding neomycin phosphotransferase type II, the gene encoding kanamycin phosphotransferase type II, the gene encoding neomycin-kanamycin phosphotransferase type II, the gene encoding hygromycin phosphotransferase, the gene encoding gentamycin acetyl transferase, the gene encoding chloramphenicol acetyltransferase, the gene encoding puromycin N-acetyl transferase (pac), the gene encoding the zeocin resistance protein or the gene encoding the bleomycin resistance protein, or a fragment, allelic variant, splice variant or mutein thereof. The (biological) activity of peptides encoded by these resistance genes, is their capability of conferring resistance to the above mentioned antibiotics.

More preferably, the inventive fusion protein comprises as a first component a peptide conferring a resistance for an antibiotic selected from:
- a puromycin N-acetyltransferase according to SEQ ID NO: 5 as encoded by the puromycin N-acetyltransferase resistance gene according to SEQ ID NO: 4;
- a neomycin phosphotransferase type II according to SEQ ID NO: 7 as encoded by the neomycin resistance gene according to SEQ ID NO: 6;
- a kanamycin phosphotransferase type II according to SEQ ID NO: 9 as encoded by the kanamycin resistance gene according to SEQ ID NO: 8;
- a neomycin-kanamycin phosphotransferase type II according to SEQ ID NO: 11 as encoded by the neomycin-kanamycin resistance gene according to SEQ ID NO: 10;
- a hygromycin phosphotransferase according to SEQ ID NO: 13 as encoded by the hygromycin resistance gene according to SEQ ID NO: 12;
- a gentamycin acetyltransferase according to SEQ ID NO: 15 as encoded by the gentamycin resistance gene according to SEQ ID NO: 14;
- a chloramphenicol acetyltransferase according to SEQ ID NO: 17 as encoded by the chloramphenicol resistance gene according to SEQ ID NO: 16;
- a zeozin resistance protein according to SEQ ID NO: 19 as encoded by the zeocin resistance gene according to SEQ ID NO: 18; and/or
- a bleomycin resistance protein according to SEQ ID NO: 21 as encoded by the bleomycin resistance gene according to SEQ ID NO: 20.

More preferably, the inventive fusion protein comprises as a first component a puromycin-N-acetyltransferase. As mentioned above, the (biological) activity of puromycin-N-acetyltransferase according to the present invention is its capability of conferring resistance to puromycin. Puromycin (puromycin dihydrochloride [3'(α-Amino-p-methoxyhydrocinnamamido)-3'-deoxy-N,N-dimethyladenosine.2HCl], C₂₂H₂₉N₇O₅.2HCl, MW.: 544.43 (Sambrook, J., Fritsch, E.F. & Maniatis, T.; Molecular Cloning: A Laboratory Manual, 3rd Edition. Cold Spring Harbor Laboratory Press, Cold Spring Harbor, New York)) is an aminonucleoside antibiotic from Streptomyces alboniger. It is an analogon to aminoacyl-tRNA and inhibits the protein synthesis by termination of the peptidyl transfer at the ribosomes in prokaryotes and eukaryotes. The antibiotic inhibits the growth of gram positive bacteria and different animal cells. Fungi and gram negative bacterias are resistant, since puromycin cannot pass the cell wall. Stock concentrations of puromycin are typically 5-50 mg/ml in dH₂O, store at -20°C, the working concentrations are typically 1-30 µg/ml (mammalian cell).

Even more preferably, the puromycin N-acetyl transferase (pac) to be used as a first component of the inventive fusion protein is a native sequence from microorganisms, preferably derived from a Streptomyces species such as *Streptomyces alboniger* or *Streptomyces coelicolor.* Preferably, the puromycin N-acetyl transferase (pac) of the inventive fusion protein is a native full-length sequence, more preferably, a native full-length sequence derived from *Streptomyces alboniger* pac*.* In a more preferred embodiment, the puromycin N-acetyl transferase (pac) of the inventive fusion protein comprises a peptide sequence according to SEQ ID NO: 5 or a peptide sequence encoded by SEQ ID NO: 4. Even more preferably, the puromycin N-acetyl transferase (pac) of the inventive fusion protein comprises amino acids 2 to 199 of SEQ ID NO: 5 or a peptide as encoded by nucleotides 3 to 597 according to SEQ ID NO: 4. Native puromycin N-acetyltransferases also encompass all naturally occurring splice variants. A "splice variant" of the puromycin N-acetyl transferase (pac) as defined above shall be understood as a puromycin N-acetyl transferase obtained by different, non-canonical splicing of the unspliced peptide of native puromycin N-acetyl transferase (pac) as defined above. More preferably, such a splice variant of the puromycin N-acetyl transferase (pac) still exhibits puromycin N-acetyl transferase (pac)-activity.

In one alternative embodiment, the inventive fusion protein comprises as a first component a fragment of a peptide conferring a resistance to an antibiotic as defined above. According to the present invention a fragment of an such a peptide is defined as a sequence having at least 50%, more preferably at least 60%, and still more preferably at least 70%, 80%, 90%, 95%, 96%, 97%, 98% or 99% sequence identity with its corresponding native peptide, wherein these fragments still confer resistance to their corresponding antibiotics (functionally active).

Alternatively or additionally, the first component of the inventive fusion protein (or the inventive fusion protein or a protein of interest as defined below) may correspond to a biologically active fragment of at least 50, 100 or 150 amino acids of its native full-length form, i.e. the native full-length form of the peptide conferring resistance to an antibiotic as defined above (or the inventive fusion protein or a protein of interest as defined below). Importantly, this fragment is still biologically active and confers resistance to an antibiotic as defined above. The (biological) activity of the first component can for example be measured by routine methods as known to a skilled person.

In still another embodiment, the first component of the inventive fusion protein comprises allelic variants of a peptide conferring resistance to an antibiotic as defined above. According to the present invention an "allelic variant" shall be understood as an alteration in the native sequence of the native form of the first component as defined above, wherein the altered sequence still confers resistance to the corresponding antibiotic. More preferably, an allelic variant of the first component as defined above has at least 50%, more preferably at least 60%, and still more preferably at least 70%, 80%, 90%, 95%, 96%, 97%, 98% or 99% sequence identity with the native form of the first component, more preferably with a sequence as defined above, e.g. SEQ ID NO: 5, more preferably with amino acids 2 to 199 of SEQ ID NO: 5, or with a sequence according to SEQ ID NOs: 7, 9, 11, 13, 15, 17, 19 or 21. The allelic variants of the first component, i.e. allelic variants of a peptide conferring resistance to an antibiotic, still confer resistance to their corresponding antibiotic, i.e. neomycin, kanamycin, neomycin-kanamycin, hygromycin, gentamycin, chloramphenicol, puromycin, zeocin or bleomycin.

The (biological) activity of the first component, i.e. conferring resistance to its corresponding antibiotic, may also be conferred by a mutein of the first component. As used herein, the term "mutein" refers to an analog of a naturally occurring polypeptide, e.g. an analog of the native form of the first component as defined above, in particular an analog of the sequences 5, 7, 9, 11, 13, 15, 17, 19 and 21 (or the inventive fusion protein or a protein of interest as defined below), in which one or more of the amino acid residues of the naturally occurring polypeptide are replaced by different amino acid residues, or are deleted, or one or more amino acid residues are added to the naturally occurring sequence of the polypeptide, without considerably lowering the activity of the resulting products as compared with the naturally occurring polypeptide. These muteins are prepared by known synthesis and/or by site-directed mutagenesis techniques, or any other known technique suitable therefore. Muteins of the first component as defined above (or of the inventive fusion protein or of a protein of interest as defined below) that can be used in accordance with the present invention, or nucleic acids encoding these muteins, preferably include a finite set of substantially corresponding sequences as substitution polypeptides or polynucleotides which can be routinely obtained by one of ordinary skill in the art, without undue experimentation, based on the teachings and guidance presented herein.

Muteins of the first component as defined above (or the inventive fusion protein or of a protein of interest as defined below) in accordance with the present invention preferably include proteins encoded by a nucleic acid, such as DNA or RNA, which hybridizes to DNA or RNA, which encode the (native form of the) first component as defined above, under moderately or highly stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., 6.3 and 6.4 (1987, 1992), and Sambrook et al. (Sambrook, J. C., Fritsch, E. F., and Maniatis, T. (2001) Molecular Cloning: A Laboratory Manual, Cold Spring Harbor Laboratory Press, Cold Spring Harbor, NY).

Without limitation, examples of stringent conditions include washing conditions at 12-20°C below the calculated Tₘ of the hybrid under study in, e.g., 2 x SSC and 0.5% SDS for 5 minutes, 2 x SSC and 0.1% SDS for 15 minutes; 0.1 x SSC and 0.5% SDS at 37°C for 30-60 minutes and then, 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC.

Muteins of the first component as defined above (or of the inventive fusion protein or of a protein of interest as defined below) include polypeptides having an amino acid sequence being at least 50% identical, more preferably at least 60% identical, and still more preferably at least 70%, 80%, 90%, 95%, 96%, 97%, 98% or 99% identical to their native form, e.g. the native form of the first component, wherein these muteins of the first component still confer resistance to an antibiotic as defined above.

A polypeptide having an amino acid sequence being at least, for example, 95% "identical" to a query amino acid sequence of the present invention, is intended to mean that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence of at least 95% identity to a query amino acid sequence, up to 5% (5 of 100) of the amino acid residues in the subject sequence may be inserted, deleted, or substituted with another amino acid.

For sequences without exact correspondence, a "% identity" of a first sequence may be determined with respect to a second sequence. In general, these two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux et al., 1984, Nucleic Acids Res. 12, 387-395. ), for example the programs BESTFIT and GAP, may be used to determine the % identity between two polynucleotides and the % identity and the % homology between two polypeptide sequences. BESTFIT uses the "local homology" algorithm of (Smith and Waterman (1981), J. Mol. Biol. 147, 195-197.) and finds the best single region of similarity between two sequences. Other programs for determining identity and/or similarity between sequences are also known in the art, for instance the BLAST family of programs (Altschul et al., 1990, J. Mol. Biol. 215, 403-410), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.).

Preferred changes for muteins in accordance with a fusion protein of the present invention are "conservative" substitutions. Conservative amino acid substitutions of the first component as defined above (or of the inventive fusion protein or of a protein of interest as defined below), may include synonymous amino acids within a group which have sufficiently similar physicochemical properties, so that a substitution between members of the group will preserve the biological function of the molecule (see e.g. Grantham, R. (1974), Science 185, 862-864). It is evident to the skilled person that amino acids may also be inserted and/or deleted in the (above-)defined sequences without altering their function, particularly if the insertions and/or deletions only involve a few amino acids, e.g. less than under thirty, and preferably less than ten, and do not remove or displace amino acids which are critical to functional activity, e.g. cysteine residues.

Preferably, synonymous amino acids, which are classified into the same groups and are typically exchangeable are defined in Table I. More preferably, the synonymous amino acids are defined in Table II, and even more preferably in Table III.

**TABLE I**

| Preferred Groups of Synonymous Amino Acids | |
|---|---|
| Amino Acid | Synonymous Group |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, lie, Val, Leu, Met |
| Trp | Trp |

**TABLE II**

| More Preferred Groups of Synonymous Amino Acids | |
|---|---|
| Amino Acid | Synonymous Group |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| Most Preferred Groups of Synonymous Amino Acids | |
|---|---|
| Amino Acid | Synonymous Group |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of the first component as defined above (or the inventive fusion protein or of a protein of interest as defined below) for use in the present invention include any known methods, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al) or as described in Sambrook et al. 2001, Molecular Cloning: A Laboratory Manual, Cold Spring Harbor, NY.

Preferably, a mutein of the present invention exhibits substantially the same biological activity as the naturally occurring polypeptide to which it corresponds.

As a second component the inventive fusion protein comprises at least one core sequence according to SEQ ID NO: 1 (Cys Cys Xaa Xaa Cys Cys), having a set of four cysteines at amino acid positions 1, 2, 5 and 6. The amino acids at positions 3 and 4 of SEQ ID NO: 1 may comprise any amino acid, selected from naturally occurring amino acids alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine, or from non-naturally occurring variants thereof, e.g. selenocysteine. More preferably, the amino acids at positions 3 and 4 of SEQ ID NO: 1 comprise a proline or a glycine (SEQ ID NO: 2). The inventive fusion protein may thus comprise as a second component at least one sequence comprising SEQ ID NO: 2. Even more preferably, in SEQ ID NO: 2 a proline is positioned at amino acid position 3 and a glycine is positioned at amino acid position 4. Additionally, any of SEQ ID NOs: 1 and 2 may comprise further amino acids at their N- and/or C-terminus, preferably selected from glycine. An exemplary preferred sequence, present at least once in the inventive fusion protein, is represented by SEQ ID NO: 3.

The second component as contained in the inventive fusion protein, preferably comprises a length of 6 to 50 amino acids, more preferably of 6 to 30 amino acids and even more preferably of 6 to 20 amino acids.

The fusion protein containing a peptide conferring resistance to an antibiotic as defined above, or the fragment, allelic variant, splice variant or mutein thereof, and at least one sequence comprising SEQ ID NO: 1, 2 or 3, is capable of binding to a ligand of the sequence comprising SEQ ID NO: 1, 2 or 3.

A "ligand" in the context of the present invention is preferably a compound, capable of binding to a sequence comprising SEQ ID NO: 1, 2 or 3. Preferably, such a ligand has fluorescent properties. Even more preferably, such a ligand is a fluorescein or a derivative therefrom, and most preferably, the ligand is a membrane permeable biarsenical fluorescein derivative, e.g. the membrane-permeable fluorescein derivative 4',5'-bis(1,3,2-dithioarsolan-2-yl)fluorescein, or any derivative thereof exhibiting the same binding and fluorescence properties.

The ligand itself is non-fluorescent in its unbound state, but becomes fluorescent upon binding to SEQ ID NO: 1, 2 or 3. It is to be noted, that SEQ ID NO: 1 represents the generic core sequence the ligand requires for binding. However, the core sequence of SEQ ID NO: 1 may be amenable to various specific variants, which are covered by the core sequence as disclosed above. The fluorescence of the ligand in its bound state may be detected using any known fluorescence detection method being suitable for detecting fluorescence signals. Preferred methods include specific generation of fluorescence signals, i.e. exciting fluorescence of the ligand with a defined wavelength, and detecting the generated fluorescence signals subsequently. Simultaneous or time-staggered generation and detection of fluorescence signals of The ligand is encompassed by this invention as well. Preferably, the fluorescence detection is carried out with a laser-induced fluorescence detection (LIF), a laser-induced time-staggered fluorescence detection (LI2F), a Fluorescence Lifetime Imaging Microscopy (FLIM), spectrophotometry, flow cytometry, white fluid fluorescence spectroscopy, or Fluorescence-Activated Cell Sorting (FACS).

Fusing as the first component a peptide conferring resistance to an antibiotic as defined above to at least one sequence according to SEQ ID NO: 1, 2 or 3, to 2, 3 or even more sequences according to SEQ ID NO: 1, 2 or 3, may lead to a fusion protein, which exhibits a stronger fluorescence signal upon binding to the ligand than a fusion protein carrying just one sequence according to SEQ ID NO: 1, 2 or 3.

A tagging of more than one of the above-defined ligand binding sequences may be used e.g. for increasing the signal/noise rate, if low fluorescence signals are to be expected, e.g. if other fluorescent components are also present in the probe.

If the first component of the inventive fusion protein or a variant thereof is fused to just one ligand binding sequence comprising SEQ ID NO: 1, 2 or 3 as second component of the inventive fusion protein, the 3' terminus of the first component, or a fragment, allelic variant, splice variant or mutein thereof, may be linked to the 5' terminus of a ligand binding sequence comprising SEQ ID NO: 1, 2 or 3, or, preferably, the 3' terminus of ligand binding sequence comprising SEQ ID NO: 1, 2 or 3 may be fused to the 5' terminus of the first component or a fragment, allelic variant, splice variant or mutein thereof.

Alternatively, if a first component as defined above or a variant thereof and more than one ligand binding sequence comprising SEQ ID NO: 1, 2 or 3 are contained in the inventive fusion protein, the ligand binding sequence comprising SEQ ID NO: 1, 2 or 3 may be positioned blockwise at the 3' terminus of the first component, or a fragment, allelic variant, splice variant or mutein thereof, via the 5' terminus of a ligand binding sequence comprising SEQ ID NO: 1, 2 or 3, and vice versa. In another alternative, two or more ligand binding sequences comprising SEQ ID NO: 1, 2 or 3 may be present at either terminus of the sequence of the first component.

The inventive fusion protein may contain a linker, which spatially separates its afore disclosed first and second component(s). Alternatively (or additionally), such a linker may be used to spatially separate the ligand binding sequences comprising SEQ ID NO: 1, 2 or 3, if a plurality of them is present in the inventive fusion protein. Typically, such a linker is an oligo- or polypeptide. Preferably, the linker has a length of 1-20 amino acids, more preferably a length of 1 to 10 amino acids and most preferably a length of 1 to 5 amino acids. Advantageously, the fusion according to the present invention comprises a linker without secondary structure forming properties, i.e. without an -helix or a -sheet structure forming tendency.
More preferably, the linker is composed of at least 50 % of glycin and/or proline residues. Most preferably, the linker is exclusively composed of glycin residues.

The inventive fusion protein or rather its components as defined above (or the protein of interest as defined below), may additionally be labelled for further detection. Such a label is preferably selected from the group of labels comprising:
(i) radioactive labels, i.e. radioactive phosphorylation or a radioactive label with sulphur, hydrogen, carbon, nitrogen, etc.
(ii) coloured dyes (e.g. digoxygenin, etc.)
(iii) fluorescent groups (e.g. fluorescein, etc.)
(iv) chemoluminescent groups,
(v) groups for immobilisation on a solid phase (e.g. His-tag, biotin, strep-tag, flag-tag, antibodies, antigene, etc.) and
(vi) a combination of labels of two or more of the labels mentioned under (i) to (v).

In a particularly preferred embodiment, the inventive fusion protein comprises the sequence according to SEQ ID NO: 23 or is encoded by the sequence according to SEQ ID NO: 22.

A second aspect of the present invention refers to nucleic acids, encoding the fusion protein as defined above. An inventive nucleic acid encoding the inventive fusion protein may comprise mRNA, RNA, genomic DNA, subgenomic DNA, cDNA, synthetic DNA, and/or combinations thereof. An inventive nucleic acid also includes any nucleic sequence variant encoding the desired amino acid sequence of an inventive fusion protein (due to degeneration of the genetic code). E.g. these alternative nucleic acid sequences may lead to an improved expression of the encoded fusion protein in a selected host organism. Tables for appropriately adjusting a nucleic acid sequence are known to a skilled person. Preparation and purification of such nucleic acids and/or derivatives are usually carried out by standard procedures (see Sambrook et al. 2001, Molecular Cloning: A Laboratory Manual, Cold Spring Harbor, NY).

A third aspect of the present invention refers to an (expression) vector. The term "vector" is used herein to designate either circular or linear DNA or RNA, which is either double-stranded or single-stranded, and which comprises at least one inventive nucleic acid to be transferred into a cell host or into a unicellular or multicellular host organism. The inventive vector comprises an inventive nucleic acid encoding the inventive fusion protein as defined above and a nucleic acid encoding a protein of interest (POI) or a mutein thereof.

A protein of interest according to the present invention may be any polypeptide the production of which is desired. The protein of interest may be applied in the field of pharmaceutics, agribusiness or furniture for research laboratories. Preferred proteins of interests find use in the field of pharmaceutics. For example, the protein of interest may be, e.g., a naturally secreted protein, a cytoplasmic protein, a transmembrane protein, or a human or a humanized antibody. When the protein of interest is a cytoplasmic or a transmembrane protein, the protein has preferably been altered such as to become soluble. Such an alteration may be carried out by any method known to a skilled person. Preferably, such an alteration is carried out e.g. by increasing the number of codons encoding hydrophilic amino acids in the coding nucleic acid sequence, e.g. by (conservatively) substituting and/or deleting nucleotides of codons encoding lipophilic and/or amphiphilic amino acids. Substitutions in the encoding nucleic acid preferably lead to amino acid substitutions as indicated in any of Tables I to III.

The polypeptide of interest may be of any origin. Preferred polypeptides of interest are of human origin and are selected e.g. from (poly)peptide hormones, cytokines, proteins involved in the blood clotting system, growth factors and factors involved in hematopoiesis.

Preferably, the protein of interest is selected from the group consisting of chorionic gonadotropin, follicle-stimulating hormone, lutropin-choriogonadotropic hormone, thyroid stimulating hormone, human growth hormone, interferons (e.g., interferon beta-1 a, interferon beta-1 b), interferon receptors (e.g., interferon gamma receptor), TNF receptors p55 and p75, interleukins (e.g., interleukin-2, interleukin-11), interleukin binding proteins (e.g., interleukin-18 binding protein), anti-CD11a antibodies, erythropoietin, granulocyte colony stimulating factor, granulocyte-macrophage colony-stimulating factor, pituitary peptide hormones, menopausal gonadotropin, insulin-like growth factors (e.g., somatomedin-C), keratinocyte growth factor, glial cell line-derived neurotrophic factor, thrombomodulin, basic fibroblast growth factor, insulin, Factor VII, Factor VIII, Factor IX, somatropin, bone morphogenetic protein-2, protein-3, protein-4, protein-5, protein-6, protein-7, protein-8, protein-9, protein-10, platelet-derived growth factor, hirudin, erythropoietin, recombinant LFA-3/IgG1 fusion protein, glucocerebrosidase, and muteins, fragments, soluble forms, functional derivatives, fusion proteins thereof, wherein a "mutein" of a protein of interest according to the present invention is as defined above in the general definition for "muteins".

In a further preferred embodiment, the protein of interest may be labeled for further detection using any of the labels as defined above. Methods for introducing such a label into the protein of interest are known to a skilled person and are described e.g. in Sambrook, J. C., Fritsch, E. F., and Maniatis, T. (2001) Molecular Cloning: A Laboratory Manual, Cold Spring Harbor Laboratory Press, Cold Spring Harbor, NY Preferably, the inventive vector is an expression vector. An "expression vector" according to the present invention preferably comprises a vector as defined above and additionally appropriate elements as expression support including various regulatory elements, such as enhancers/promoters from viral, bacterial, plant, mammalian, and other eukaryotic sources that drive expression of the inserted polynucleotide in host cells, such as insulators, boundary elements, LCRs (e.g. described by Blackwood and Kadonaga (1998), Science 281, 61-63) or matrix/scaffold attachment regions (e.g. described by Li, Harju and Peterson, (1999), Trends Genet. 15, 403-408).

The term "promoter" as used herein refers to a region of DNA that functions to control the transcription of one or more DNA sequences, and that is structurally identified by the presence of a binding site for DNA-dependent RNA-polymerase and of other DNA sequences, which interact to regulate promoter function. A functional expression promoting fragment of a promoter is a shortened or truncated promoter sequence retaining the activity as a promoter. Promoter activity may be measured by any assay known in the art, e.g. by a reporter assay using luciferase as reporter gene (Wood, de Wet, Dewji, and DeLuca, (1984), Biochem Biophys. Res. Commun. 124, 592-596; Seliger and McElroy, (1960), Arch. Biochem. Biophys. 88, 136-141) or commercially available from Promega^{®}).

In a preferred embodiment, the inventive expression vector comprises at least one promoter of the murine CMV immediate early region. The promoter may for example be the promoter of the mCMV IE1 gene (the "IE1 promoter"), which is known from, e.g. WO 87/03905. The promoter may also be the promoter of the mCMV IE2 gene (the "IE2 promoter"), the mCMV IE2 gene itself being known from, e.g., Messerle, Keil, and Koszinowski. 1991, J. Virol. 65, 1638-1643. The IE2 promoter and the IE2 enhancer regions are described in details in PCT/EP2004/050280.

An "enhancer region" as used in the inventive expression vector, typically refers to a region of DNA that functions to increase the transcription of one or more genes. More specifically, the term "enhancer", as used herein, is a DNA regulatory element that enhances, augments, improves, or ameliorates expression of a gene irrespective of its location and orientation vis-à-vis the gene to be expressed, and may be enhancing, augmenting, improving, or ameliorating expression of more than one promoter.

Additionally, the inventive expression vector may comprise an amplification marker. This amplification marker may be selected from the group consisting of, e.g. adenosine deaminase (ADA), dihydrofolate reductase (DHFR), multiple drug resistance gene (MDR), ornithine decarboxylase (ODC) and N-(phosphonacetyl) -L-aspartate resistance (CAD). Amplification of the gene encoding the above defined proteins, i.e. the protein of interest (POI) and/or the inventive fusion protein, allows to increase the expression level of these proteins upon integration of the vector in a cell (Kaufman et al. (1985), Mol. Cell Biol. 5, 1750-1759).

According to one embodiment, the inventive expression vector comprises one promoter or a promoter assembly, wherein this promoter or promoter assembly drives the expression of both the protein of interest (POI) or a mutein thereof, and the inventive fusion protein. Therefore, the protein of interest and the inventive fusion protein are preferably contained "in frame" in one expression cassette in the inventive expression vector, wherein the coding regions of both are separated by an internal ribosomal entry site (IRES), thus forming a bicistronic nucleic acid sequence in the inventive vector. Such a (internal ribosomal entry site) sequence allows the ribosomal machinery to initiate translation from a secondary site within a single transcript and thus to express both the protein of interest and the inventive fusion protein as two separate proteins, when using just one promoter/promoter assembly. This embodiment ensures an optimal correlation between expression of the inventive fusion protein and expression of the POI. Such correlation is essential, when using the inventive fusion protein for screening cells for high expression of a POI.
Alternatively, the inventive expression vector may comprise at least two promoters or promoter assemblies, wherein one of these promoters drives the expression of the inventive fusion protein, and the other one drives the expression of the protein of interest (POI). In this embodiment, the expression vector preferably carries two expression cassettes, the first carrying the inventive fusion protein and the second one the protein of interest, wherein each expression cassette is functionally linked with a promoter and/or enhancer sequence as defined above. Accordingly, this embodiment does not produce just one transcript including both the protein of interest and the inventive fusion protein linked by an IRES sequence. Instead, two transcripts are provided. Such a system may be advantageously used, if the molecular weight of the protein of interest exceeds a critical value. In a preferred embodiment of this alternative, the promoters of the murine CMV immediate early region regulate the expression of genes encoding the protein of interest, and the inventive fusion protein is expressed from an additional expression cassette inserted in the vector backbone. The mCMV(IE1) and mCMV(IE2) promoters may regulate the expression either of two identical copies of the gene encoding the protein of interest, or of two subunits of a multimeric protein of interest such as antibodies or peptide hormones.

A fourth aspect of the invention refers to host cells transfected with an inventive (expression) vector. Many cells are suitable for such a transfection in accordance with the present invention, e.g. primary or established cell lines from a wide variety of eukaryotes including plant, yeast, human and animal cells, as well as prokaryotic, viral, or bacterial cells. Preferably, inventive host cells are eukaryotic cells, derived e.g. from eukaryotic microorganisms, such as Saccharomyces cerevisiae (Stinchcomb et al., Nature, 282:39, (1997)). More preferably, cells from multi-cellular organisms are selected as host cells for expression of nucleic acid sequences according to the present invention. Cells from multi-cellular organisms are particularly preferred, if post-translational modifications, e.g. glycosylation of the encoded proteins, are required (N and/or O coupled). In contrast to prokaryotic cells, higher eukaryotic cells may permit these modifications to occur. The skilled person is aware of a plurality of established cell lines suitable for this purpose, e.g. 293T (embryonic kidney cell line), HeLa (human cervix carcinoma cells) and further cell lines, in particular cell lines established for laboratory use, such as HEK293-, Sf9- or COS-cells or cells of the immune system or adult stem cells, such as stem cells of the hematopoietic system (derived from bone marrow). More preferably, the cell is a mammalian cell. Most preferably, said cell is a cell from Chinese hamster or a human cell. For example, suitable cells include NIH-3T3 cells, COS cells, MRC-5 cells, BHK cells, VERO cells, CHO cells, rCHO-tPA cells, rCHO - Hep B Surface Antigen cells, HEK 293 cells, rHEK 293 cells, rC127 - Hep B Surface Antigen cells, CV1 cells, mouse L cells, HT1080 cells, LM cells, Yl cells, NSO and SP2/0 mouse hybridoma cells and the like, RPMI-8226 cells, Vero cells, WI-38 cells, MRC-5 cells, Normal Human fibroblast cells, Human stroma cells, Human hepatocyte cells, human osteosarcoma cells, Namalwa cells, human retinoblast cells, PER.C6 cells and other immortalized and/or transformed mammalian cells.

A fifth aspect of the present invention refers to a method of screening cells for expression or high expression of a protein of interest, said method comprising the steps of:
(i) transfecting cells with an inventive expression vector;
(ii) selecting cell clones being resistant to an antibiotic as defined above;
(iii) incubating cells selected according to step (ii) with a solution containing the ligand; and
(iv) detecting the fluorescence activity of cell clones selected according to step (ii) due to fluorescence of the ligand.

In step (i) of the inventive cell screening method of screening cells, cells are transfected with an inventive expression vector as defined above. Therefore, the cells to be transfected in step (i) are preferably cells, which upon successful transfection, should express both the inventive fusion protein and the protein of interest (POI). More preferably, cells to be transfected are selected from the cell lines disclosed above. The transfection may be performed by methods known to a skilled person and as described in the prior art, e.g. Sambrook, J. C., Fritsch, E. F., and Maniatis, T. (2001) Molecular Cloning: A Laboratory Manual, Cold Spring Harbor Laboratory Press, Cold Spring Harbor, NY.

In step (ii) of the inventive cell screening method, cells are selected which are resistant to an antibiotic as defined above, i.e. which were successfully transfected in step (i) and express a peptide conferring a resistance for an antibiotic as defined above (i.e. neomycin, kanamycin, neomycin-kanamycin, hygromycin, gentamycin, chloramphenicol, puromycin, zeocin or bleomycin, respectively). Accordingly, cells are preferably grown, typically for 1 hour up to 3 weeks, in a culture medium under selective conditions, i.e. in the presence of the corresponding antibiotic for exerting selection pressure from the very beginning of cultivation. Alternatively, cells are typically grown for 1 hour up to 3 weeks, in a culture medium under non-selective conditions, and the corresponding antibiotic is preferably added at a predetermined time, e.g. when cells exhibit a specific optical density (OD-value). Suitable cell culturing conditions are preferably those known to a skilled person and as described in the prior art, e.g. Sambrook, J. C., Fritsch, E. F., and Maniatis, T. (2001) Molecular Cloning: A Laboratory Manual, Cold Spring Harbor Laboratory Press, Cold Spring Harbor, NY.

In subsequent step (iii) cells as selected in step (ii) are typically incubated with a solution containing a membrane-permeable fluorescein derivative 4',5'-bis(1,3,2-dithioarsolan-2-yl)-fluorescein, or any derivative thereof exhibiting the same binding properties. Thereby the inventive fusion protein is labeled with the ligand (or a derivative thereof) upon binding to its component(s), comprising at least one sequence SEQ ID NO: 1, 2 or 3. Labeling with the ligand may be performed by using the labeling protocol according to Example 2 (see below). Alternatively, the Lumio™In-Cell Labeling Kit from Invitrogen Corporation may be used according to the manufacturers instructions. Similarly, labeling with a derivative of the ligand may be performed according to these protocols.

In final step (iv) fluorescence of the labelled cells is elicited via the acquired fluorescence of the ligand, or a derivative thereof. Fluorescence of the ligand, when bound to any of SEQ ID NO: 1, 2 or 3 of the inventive fusion protein, may be evoked after excitation. The emitted fluorescence spectra can be detected by using any of the above mentioned methods for detecting fluorescence, most preferably by using FACS. The excitation wavelength is typically in a range from 450 to 650 nm and emittance of fluorescent light is typically observed in a range of from 450 to 700 nm.

Any number of cells may be screened by such a method. Preferably, the fluorescence activity of at least 20, 50, 100, 500, 1.000, 5.000, 10.000, 50.000, 100.000, 500.000 or 1.000.000 cells is detected in step (iv). Most preferably, a population of cells sufficient for obtaining at least 1.000 to 10.000.000 independent transfectants being resistant to an antibiotic as defined above is screened. Among these, at least 10 to 1.000.000 candidate clones being resistant to this antibiotic may be sorted by evaluating the fluorescence activity of these cells. Preferably, about 20% of cells that exhibit highest fluorescence activity in step (iv) are selected as cells that exhibit highest expression of said protein of interest. More preferably, the 10% of cells that exhibit highest fluorescence activity in step (iv) comprise the cells that exhibit highest expression of said protein of interest. Even more preferably, the 5% of cells that exhibit highest fluorescence activity in step (iv) comprise the cell that exhibit highest expression of said protein of interest. Preferably, the cells are screened cell by cell using FACS.

In the context of the present invention, "high expression" refers to an expression level in a cell, which is screened, that is higher than in other cells that are screened. "High expression" of a protein is a relative value. For example, final expression levels of recombinant proteins that are commercially produced range from 1 to 2.000 mg/l (cell culture), depending on the protein, annual quantities required and therapeutic dose. During a screening, the expression level of a protein of interest is typically lower than the final expression level.

The cells obtained at the end of the above screening method may be ranked relative to each other regarding the expression level of the protein of interest (POI). Particularly, the cells exhibiting the highest fluorescence activity may be selected at the end of the above method of screening. For example, individual cells exhibiting fluorescence activity corresponding to the top 5-20% of inventive expressors are selected for further analysis of expression of the gene of interest in a subsequent step.

In a preferred embodiment, the above screening method further comprises an optional step (v) comprising selecting about 5% to about 20% of the cells assayed in step (iv), wherein the selected cells are those exhibiting highest fluorescence activity in step (iv). Alternatively, about 5% to about 30%, 40%, 50%, 60%, 70% or 80% of the cells assayed in step (iv) may be selected based on highest activity of the protein of interest. Then, upon selection of the cells exhibiting the highest fluorescence activity, the expression level of the protein of interest in said selected cells may further be determined.

In another preferred embodiment, the above method of screening is performed using multiwell microtiter plates or similar.

A sixth aspect of the present invention refers to a method for obtaining a cell line expressing a protein of interest, said method comprising the step of:
(i) screening cells according to any of the above inventive cell screening methods;
(ii) selecting the cell(s) exhibiting the highest expression of said protein of interest, preferably according to any of the above inventive methods; and
(iii) establishing a cell line from said cell.

As used herein, a "cell line" refers to one specific type of cell that can grow in a laboratory, i.e. cell lines from cells as defined above. A cell line can usually be grown in a permanently established cell culture, and will proliferate indefinitely given appropriate fresh medium and space. Methods of establishing cell lines from isolated cells are well-known by those of skill in the art. Preferably, cell lines are prepared from cells as mentioned above.

A seventh aspect refers to a method of producing a protein of interest, said method comprising the steps of:
(i) culturing a cell line obtained according to an inventive method as described above, under conditions which (selectively) permit expression of said protein of interest; and
(ii) isolating said protein of interest.

Conditions which (selectively) permit expression of the protein of interest can easily be established by one of skill in the art by standard methods. Alternatively, any condition suitable for the protein of interest to be expressed and known to a skilled person may be used. Such methods are disclosed in e.g. Sambrook, J. C., Fritsch, E. F., and Maniatis, T. (2001) Molecular Cloning: A Laboratory Manual, Cold Spring Harbor Laboratory Press, Cold Spring Harbor, NY.

In the context of the present invention "isolating" typically comprises purifying the protein of interest. The purification may be carried out by any technique well-known by those of skill in the art, e.g. by conventional biochemical methods, such as chromatography, e.g. affinity chromatography (HPLC, FPLC, ...), size exclusion chromatography, etc., as well as by cell sorting assays, antibody detection, etc.. or by any method disclosed by Sambrook et al, (2001, supra). In case the protein of interest shall be applied as medicament, it is preferably formulated into a pharmaceutical composition. Preferably, such pharmaceutical compositions comprises the protein of interest as disclosed above. Additionally, such a pharmaceutical composition may comprise a pharmaceutically acceptable carrier, adjuvant, or vehicle according to the invention refers to a non-toxic carrier, adjuvant or vehicle that does not destroy the pharmacological activity of the protein of interest with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Furthermore, an eighth aspect of the present invention refers to a method of producing an inventive fusion protein comprising the steps of:
(i) culturing a cell as defined above (e.g. comprising a nucleic acid encoding the inventive fusion protein) under conditions which (selectively) permit expression of the inventive fusion protein; and
(ii) isolating the inventive fusion protein.

In the context of the present invention "isolating" also comprises purifying the inventive fusion protein, if necessary. The purification may be carried out by any method as disclosed above. Furthermore, such a method may for example be performed e.g. as described in Example 1.

Such a method as disclosed above for producing an inventive fusion protein may be suitable, e.g. for, without being limited, discovering the properties of the inventive fusion protein in vitro, e.g. binding properties of the membrane permeable fluorescein derivative, signal intensity, exhibited upon binding, solubility of the fusion protein under physiologic conditions, etc..

A ninth aspect of the present invention refers to the use of a cell as disclosed above comprising an inventive nucleic acid as disclosed above for producing a protein of interest. Preferably, said inventive nucleic acid is contained in a vector or an expression vector, preferably an (expression) inventive vector as defined above.

A tenth aspect of the invention refers to the use of an inventive fusion protein as defined above, of a nucleic acid according to the present invention or of an inventive (expression) vector for screening cells for expression or for high expression of a protein of interest. Preferably, cells are therefore screened at first in a primary screen for high fluorescence activity. Then, fluorescence activity may be correlated to the expression of a protein of interest by inference. This allows to rapidly eliminate 80 to 95% of the tested cells based on low fluorescence activity, and to retain the remaining 5-20% for analysis of expression of the gene of interest in a step.

Having now fully described this invention, it will be appreciated by those skilled in the art that the same can be performed within a wide range of equivalent parameters, concentrations and conditions without departing from the spirit and scope of the invention and without undue experimentation.

While this invention has been described in connection with specific embodiments thereof, it will be understood that it is capable of further modifications. This application is intended to cover any variations, uses or adaptations of the invention following, in general, the principles of the invention and including such departures from the present disclosure as come within known or customary practice within the art to which the invention pertains and as may be applied to the essential features hereinbefore set forth as follows in the scope of the appended claims.

All references cited herein, including journal articles or abstracts, published or unpublished patent applications, issued patents or any other references, are entirely incorporated by reference herein, including all data, tables, figures and text presented in the cited references. Additionally, the entire contents of the references cited within the references cited herein are also entirely incorporated by reference. Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### BRIEF DESCRIPTION OF THE FIGURES

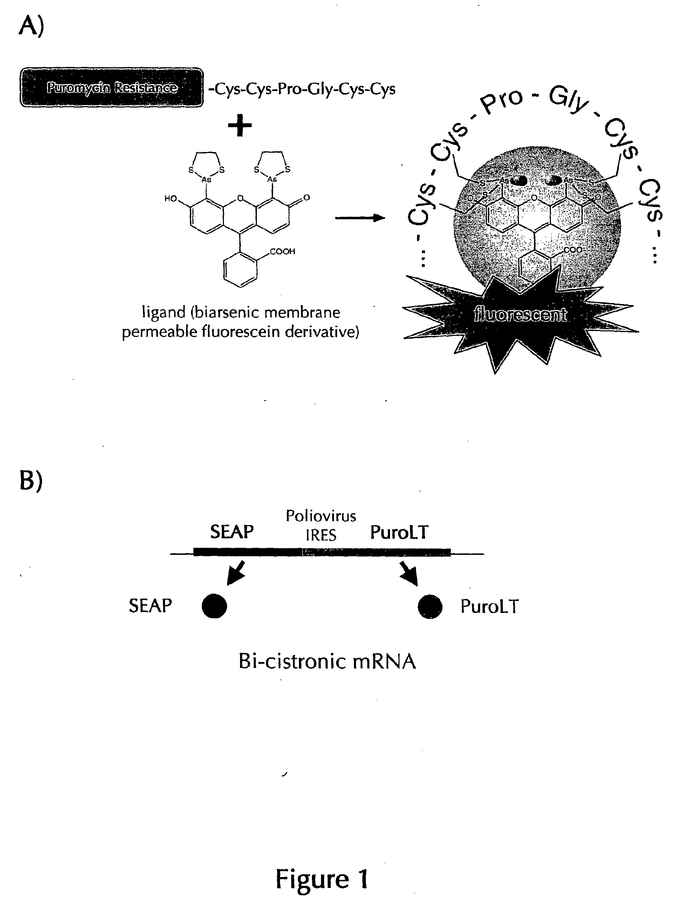

- **Figure 1**:: shows the principle of binding of a ligand to SEQ ID NO's: 1, 2 or 3 (1A) in the inventive fusion protein. Just upon binding to any of SEQ ID NO's: 1, 2 or 3, the ligand becomes fluorescent and may be detected using common fluorescence detection methods.
In Figure 1 B, an exemplary bi-cistronic mRNA, encoding the inventive fusion protein and the protein of interest, is disclosed, wherein both coding sequences are separated by an IRES sequence. Linking expression of the gene of interest (p.ex. SEAP) to the fusion protein on a bicistronic mRNA allows correlated expression of both proteins. High expression of the inventive fusion protein is thus correlated with strong fluorescence, and this is indicative for high SEAP production.

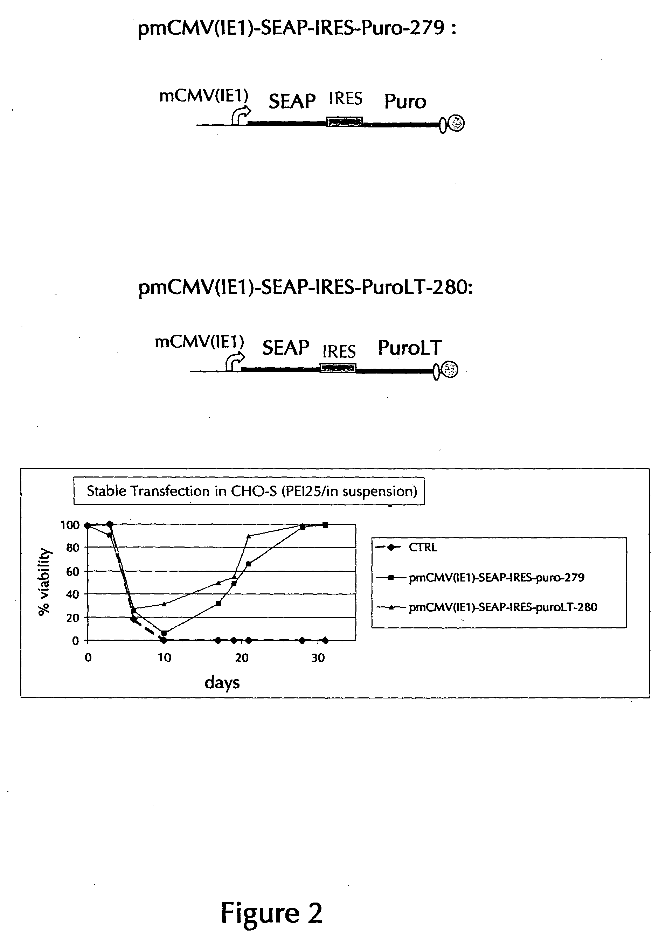

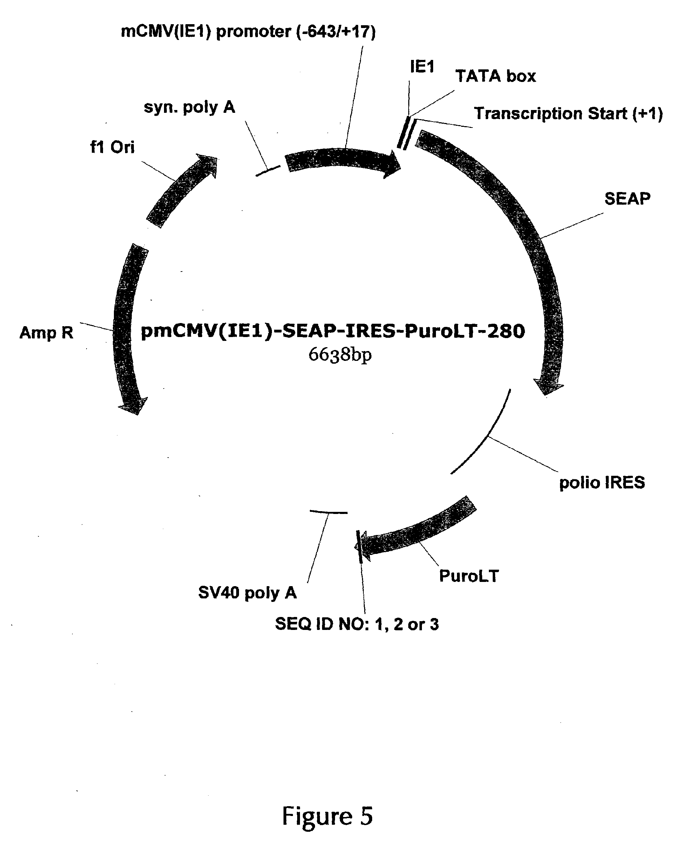

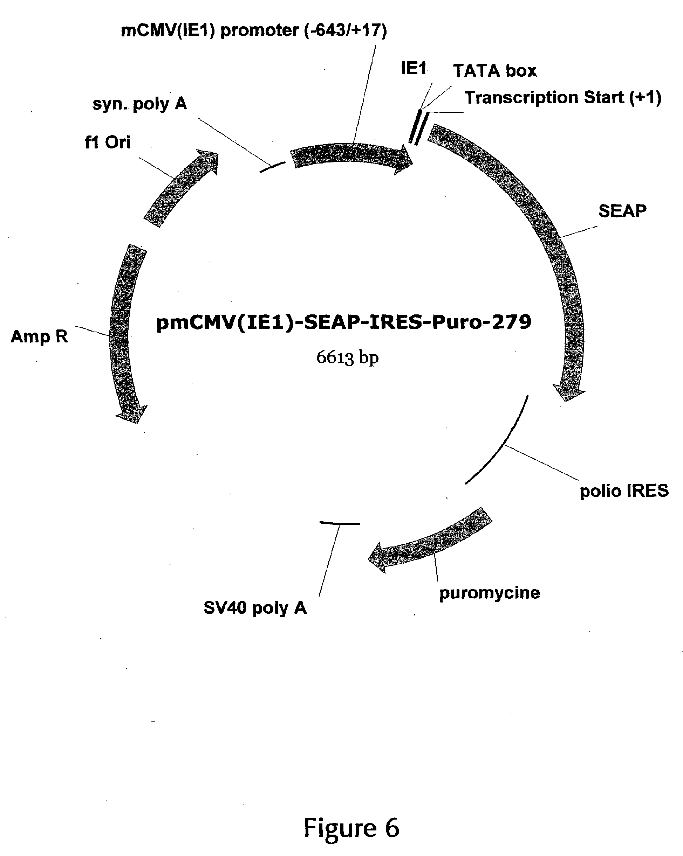

- **Figure 2:**: shows transfection of plasmids pmCMV(IE1)-SEAP-IRES-Puro-279 (in more detail disclosed in Figure 6) and pmCMV(IE1)-SEAP-IRES-PuroLT-280 (in more detail disclosed in Figure 5) into CHO-S cells (PE125 / in suspension). Selection of stable transfectants using puromycin as a first component leads to recovery of viability up to 100% after 2 or 3 weeks. In a control, wherein cells comprise a plasmid without puromycin resistance, all cells were depleted.

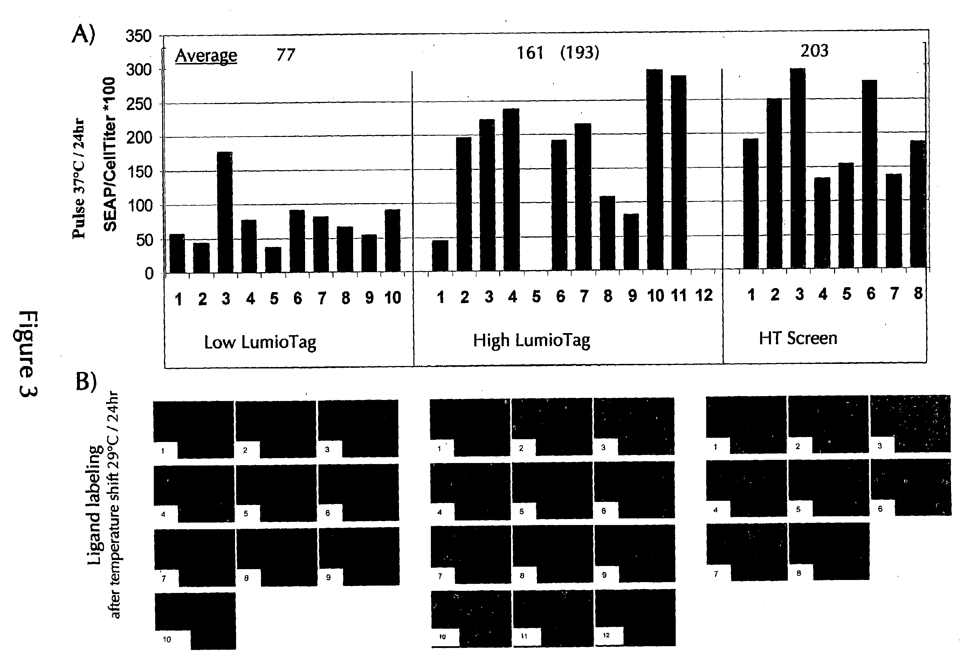

- **Figure 3:**: shows the correlation between SEAP expression levels (upper row) and fluorescence intensity subsequent to labeling with 4',5'-bis(1,3,2-dithioarsolan-2-yl)fluorescein (lower row) for thirty different clones. The left column ("Low LumioTag") and middle column ("High LumioTag") show clones screened using the inventive fusion protein as a bifunctional marker, as described in detail in Example 7. The right column ("HT Screen") shows clones screened using a classical high-throughput screening approach

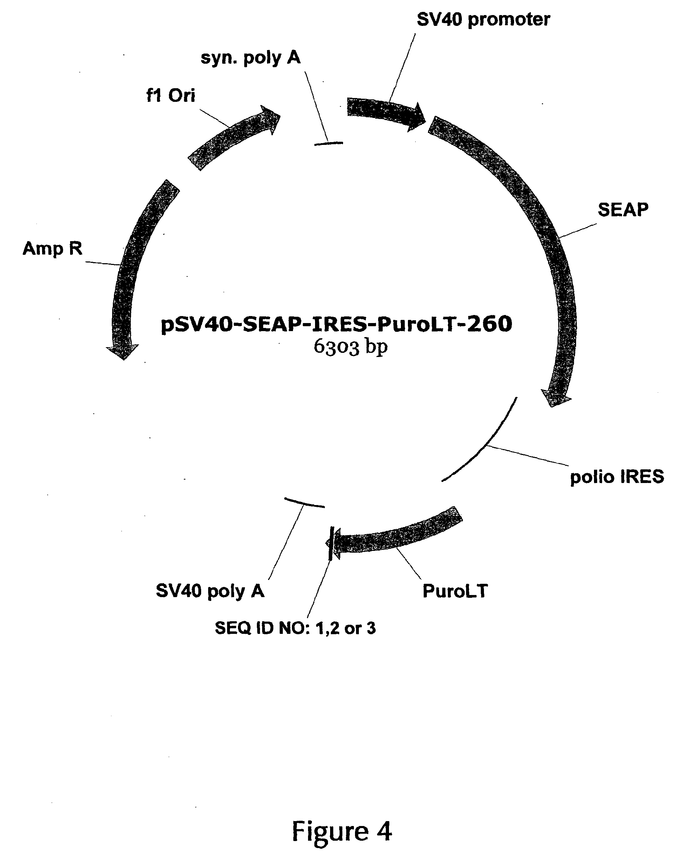

- **Figure 4**:: shows the plasmid map of pSV40-SEAP-IRES-PuroLT-260, having 6303 bp. pSV40-SEAP-IRES-PuroLT-260 comprises a SV40 promoter, a SEAP coding sequence and a sequence, coding for an exemplary inventive fusion protein (herein designated puroLT). Both coding sequences are separated by a poliovirus IRES sequence.
- **Figure 5:**: shows the plasmid map of pmCMV(IE1)-SEAP-IRES-PuroLT-280, having 6638 bp. pmCMV(IE1)-SEAP-IRES-PuroLT-280 differs from pSV40-SEAP-IRES-PuroLT-260 (Figure 4) in that the mCMV(IE1) promoter was used instead of the SV40 promoter.
- **Figure 6:**: shows the plasmid map of pmCMV(IE1)-SEAP-IRES-Puro-279, having 6613 bp. pmCMV(IE1)-SEAP-IRES-Puro-279 differs from pmCMV(IE1)-SEAP-IRES-PuroLT-280 in that the sequence encoding puromycin N-acetyl transferase was used instead of the sequence for the inventive fusion protein. pmCMV(IE1)-SEAP-IRES-Puro-279 preferably serves as a negative control in the experiments.

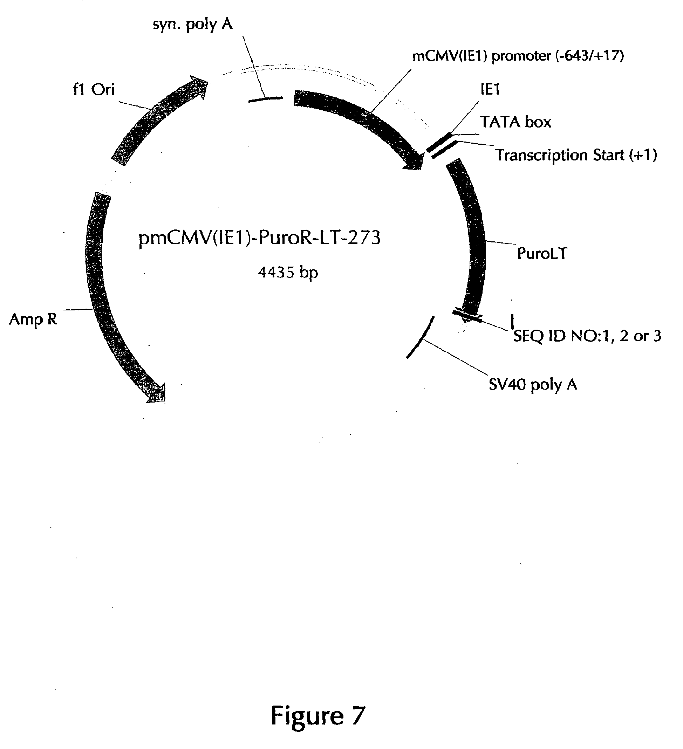

- **Figure 7:**: shows the plasmid map of pmCMV(IE1)-PuroR-LT-273, having 4435 bp. pmCMV(IE1)-PuroR-LT-273 differs from pmCMV(IE1)-SEAP-IRES-Puro-279 in that the coding sequence for SEAP and the IRES sequence are missing. pmCMV(IE1)-SEAP-IRES-Puro-279 also serves as a control in the experiments.

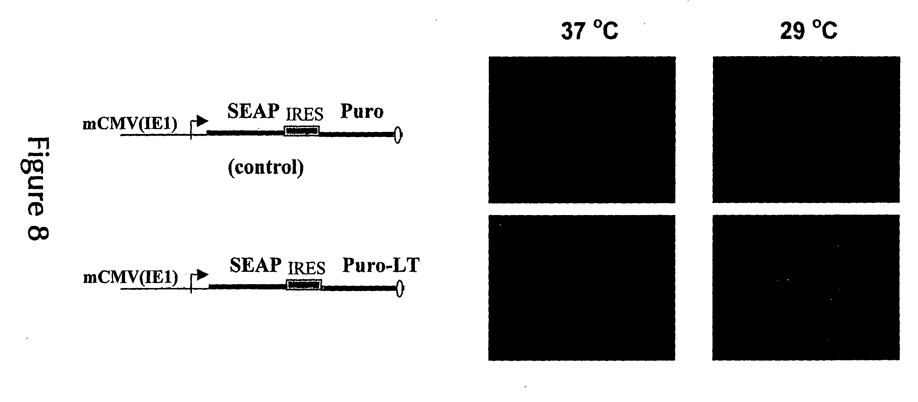

- **Figure 8**:: depicts labeling with the ligand (here 4',5'-bis(1,3,2-dithioarsolan-2-yl)fluorescein) and transient transfections in CHO cells. As may be seen from the experiments, temperature is shifted prior to staining.

Furthermore, the inventive fusion protein will be detected if the expression level is high enough. Following results were obtained for the inventive constructs:

| | at 37°C | at 29°C |
|---|---|---|
| pmCMV(IE1)-SEAP-IRES-PuroLT-279 | +++ | +++ |
| pmCMV(IE1)-SEAP-IRES-PuroLT-280 | - | ++ |

The expression level of pmCMV(IE1)-SEAP-IRES-PuroLT-279 thus showed no temperature shift. However, a temperature shift was observed for expression of pmCMV(IE1)-SEAP-IRES-PuroLT-280, comprising the inventive fusion protein with SEAP and IRES sequences. Higher PuroLT levels at 29°C in this respect could result from increased transcription or IRES activity, mRNA or protein stability. As a conclusion, the induction times from o/n to 24hr were sufficient.

### BRIEF DESCRIPTION OF THE SEQUENCES OF THE SEQUENCE LISTING

- SEQ ID NO: 1: corresponds to the generic binding sequence of the ligand of SEQ ID NOs: 1, 2 or 3.
- SEQ ID NO: 2: corresponds to a more specific binding sequence of the ligand of SEQ ID NOs: 1, 2 or 3, wherein amino acids at positions 3 and 4 in SEQ ID NO: 2 are defined as Proline and Glycine, respectively.
- SEQ ID NO: 3: corresponds to a more specific binding sequence of the ligand of SEQ ID NOs: 1, 2 or 3, which is extended N- and C-terminally with respect to SEQ ID NO: 2.
- SEQ ID NOs: 4,5: correspond to the resistance gene for the antibiotic puromycin and the encoded puromycin N-acetyltransferase of *Streptomyces alboniger* pac.
- SEQ ID NOs: 6,7: correspond to the resistance gene for the antibiotic neomycin and the encoded neomycin phosphotransferase type II.
- SEQ ID NOs: 8,9: correspond to the resistance gene for the antibiotic kanamycin and the encoded kanamycin phosphotransferase type II.
- SEQ ID NOs: 10,11: correpond to the resistance gene for the antibiotic neomycin-kanamycin and the encoded neomycin-kanamycin phosphotransferase type II.
- SEQ ID NOs: 12,13: correpond to the resistance gene for the antibiotic hygromycin and the hygromycin phospho transferase.
- SEQ ID NOs: 14,15: correpond to the resistance gene for the antibiotic gentamycin and the encoded gentamycin acetyl transferase.
- SEQ ID NOs: 16,17: correspond to the resistance gene for the antibiotic chloramphenicol and the encoded chloramphenicol acetyltransferase.
- SEQ ID NOs: 18,19: correspond to the resistance gene for the antibiotic zeocin and the encoded zeocin resistance protein.
- SEQ ID NOs: 20,21: correspond to the resistance gene for the antibiotic bleomycin and the encoded bleomycin resistance protein.
- SEQ ID NOs: 22,23: correspond to the nucleic acid sequence encoding an exemplary inventive chimeric selection marker and the inventive chimeric selection marker.
- SEQ ID NOs: 24,25: correspond to primers oSerono1206 and oSerono1239, used for constructing an exemplary inventive fusion protein.

### EXAMPLES

### 1. Example 1: Construction of an exemplary inventive fusion protein by PCR

A gene encoding the fusion protein, comprising puromycin N-acetyl transferase (pac) and SEQ ID NO: 3, and a protein of interest (here SEAP, secreted alkaline phosphatase) was constructed by fusing the open reading frame for puromycin N-acetyl transferase (pac) fused to SEQ ID NO: 3, by PCR cloning into an expression vector comprising a first open reading frame encoding SEAP, followed by a poliovirus IRES. The poliovirus IRES sequence allows separating two open reading frames, which are expressed from the same promoter but as two separate proteins..

### 1.1 Cloning of nucleic acid for pSV40-SEAP-IRES-puroLT-260

Therefore, a gene encoding a fusion of a peptide (-GCCPGCCGGG, SEQ ID NO: 3) to the C-terminus of the puromycin resistance gene was created by the polymerase chain reaction (PCR) using oligos oSerono1206 (5'-GTGGCTGCTTATGGTGACAATC-3', SEQ ID NO: 24) and oSerono1239 (5'-CGCGCTAGCTCATTACTAGCCGCCACCGCAACAGCCAGGACAACAGCCGGCA CCGGGCTTGCGGGTC-3', SEQ ID NO: 25). The resulting gene (designated PuroLT) was cloned into the pSV40-SEAP-IRES-puro-227 vector, which confers resistance to puromycin and comprises the SEAP open reading frame under the control of the SV40 promoter. The resulting plasmid was referred to as pSV40-SEAP-IRES-PuroLT-260. The inserted fragment was verified by sequencing.

The SV40 promoters of pSV40-SEAP-IRES-puro-227 and pSV40-SEAP-IRES-PuroLT-260 were replaced with the murine CMV IE1 promoter (mCMV(IE1), described e.g. in WO 87/03905) to generate pmCMV(IE1)-SEAP-IRES-Puro-279 and pmCMV(IE1)-SEAP-IRES-PuroLT-280, respectively.

### The PCR conditions were as follows:

*• Amplification of pac.* 25 pmol of primers of SEQ ID Nos. 24 and 25 were mixed with about 20ng of the Xbal/Mfel fragment from a vector comprising the pac open reading frame, 200 M of each dNTPs, 1 x KOD, 2 units of KOD DNA polymerase (KOD Hot Start DNA polymerase, catalogue No. 71086-3, Novagen). The final volume was of 100µl.
*• Cycling.*
   - First step: 3 minutes at 94°C
   - 12 cycles: (i) denaturation of 15 seconds at 94°C; (ii) hybridization of 15 seconds at 55°C; and (iii) polymerization of 1 minute at 72°C;
   - Final step: 7 minutes at 72°C

The obtained PCR product for puroLT was firstly analyzed by PAGE analysis. Each PCR reaction was purified using the QlAquick PCR purification kit (Catalog No. 28106, Qiagen) following manufacturer's protocol.

For cloning into the pSV40-SEAP-IRES-puro-227 vector, the PCR fragment was purified using the MinElute Gel Extraction kit (Catalog No. 28606, Qiagen) following manufacturer's protocol.

### 1.2 Cloning of nucleic acid for pmCMV(IE1)-SEAP-IRES-puroLT-280

Subsequent to verifying the pSV40-SEAP-IRES-puroLT-260 vector sequence (see 1.1) the SV40 promoter sequence was replaced by the murine CMV promoter to generate pmCMV(IE1)-SEAP-IRES-PuroLT-280.

### 1.3 Cloning of nucleic acid for pmCMV(IE1)-SEAP-IRES-puro-279

Cloning of nucleic acid for pmCMV(IE1)-SEAP-IRES-PuroLT-279 was carried out similar as disclosed above for pmCMV(IE1)-SEAP-IRES-PuroLT-280, wherein the nucleic acid encoding puromycin N-acetyl transferase (puro) was cloned into the vector instead of the nucleic acid encoding the inventive fusion protein.

### 2. Example 2: Labeling protocol with the ligand

The inventive fusion protein was labeled with the ligand using following protocol.
- Cells were firstly washed 1 x with HBSS (Hanks balanced salt solution, Gibco cat#14025-050). If cells were used grown in ProCHO5 - Pluronic acid at 0.05% final concentration was included.
- Cells were incubated cells in 1 x Labeling Solution for 30 minutes at room temperature in the dark. The 1 x Labeling Solution comprises HBSS (Gibco cat#14025-050). 1 M 4',5'-bis(1,3,2-dithioarsolan-2-yl)fluorescein and 50 µM EDT (1,2-Ethandithiol Sigma cat# 39,802-0). If cells were used, which were grown in ProCHO5 Pluronic acid at 0.1% final concentration was added.
- The Labeling Solution was removed (and discarded appropriately). The cells were then washed once in HBSS + 50 µM EDT. If cells were used, which were grown in ProCHO5, Pluronic acid at 0.1% final concentration was used.
- Cells were then added or resuspended in HBSS + 20 µM Disperse Blue 3 (supplied with LumioGreen Kit Invitrogen cat# 45-7510). If cells were used, which were grown in ProCHO5, Pluronic acid at 0.1% final concentration was included.

In order to detect fluorescence of a ligand to SEQ ID NO: 1, 2 or 3 in cells expressing the inventive fusion protein, the cells were pre-incubated o/n to 24hr at 29°C prior to labeling with 4',5'-bis(1,3,2-dithioarsolan-2-yl)fluorescein.

After labeling with 4',5'-bis(1,3,2-dithioarsolan-2-yl)fluorescein, the cells were observed under a fluorescence microscope (Olympus CKX41 microscope equipped with a DP50 digital camera) using a standard FITC filter set.

### 3. Example 3: Transfection of vectors

### 3.1 Transfection of vectors encoding the inventive fusion protein

One day before transfection, cells grown in ProCHO5 medium were passaged at 0.75x10⁶ cells/ml. Just before transfection, 8-10x10⁶ cells were centrifuged, washed with RPMI 1640+ Glutamax, resuspended in 15 ml of the same medium and distributed in 6w plates (2.5 ml/well) or 24w plates (0.5 ml/well).

Linear PEI25 (MW25000, Polysciences, Cat. #23966) was used as transfecting agent at 3-3.5 µl of 1 mg/ml PEI25 solution per µg of DNA. The PEI25 1 mg/ml solution was filter sterilised, aliquoted in 1 ml fractions and kept at -70°C.

Plasmid DNA in 150mM NaCl was mixed with PEI25, incubated 10 min at RT and added to the cells. After 2 hours at 37°C, transfection medium was removed and replaced by 3 ml of ProCHO5 supplemented with 4mM glutamine and 1 xHT. Plates were incubated o/n at 37°C with shaking at 60 rpm. Cells were pooled from all the wells and plated at 0.5x10⁶ cells/ml of ProCHO5 (supplemented with 4mM glutamine and 1 xHT) in P150 Petri dishes. 48 hours post-transfection cells were counted, spent medium was removed by centrifugation, and cells were then diluted to 1.0 x 10⁶ viable cells/ml in selection medium (PrOCHO5 supplemented with 4.5 mM L-glutamine, 1 xHT and 10 g/ml of puromycin). The medium was changed every other day. Cell densities were monitored over time and, when the number of viable cells dropped below 0.1 x 10⁶ cells/ml, the cells were concentrated in a smaller volume. Otherwise, when the number of viable cells increased, cells were diluted to 0.4 -0.5 x 10⁶ cells/ml. This procedure was repeated until the viability of the pool reached 90%.

The pool, selected and transfected with plasmid pmCMV(IE1)-SEAP-IRES-PuroLT-280, was seeded at 1 cell/well in 4 384 well plates in ProCHO5 medium supplemented with 4 mM glutamine, 1 xHT and 10 µg/ml of puromycin. 176 clones were recovered in 96 well plates.

### 3.2 Selection for resistance to puromycin

Cells were transferred to a 15 ml Falcon tube, centrifuged, and the cell pellet was resuspended in 2 ml medium containing 5% Fetal Bovine Serum (FBS) in a 6 well plate. Selection was applied 48 hours post transfection, by exchanging the medium for ProCHO5/HT/Glutamine/5% FBS containing 10 µg/ml of puromycin (Sigma, P-8833). Every two days, a medium exchange was performed by discarding the old medium, washing with 1 x PBS, and adding fresh selective medium. After 2 weeks of selection, the cells were trypsinized, counted, and a series of dilutions corresponding to 1000, 500, 100, 50, 20, 10 cells/well of a 6-w format was performed. Ten days later, the colonies growing in all dilutions were counted, and all of them were picked to allow growth in suspension in the absence of serum for clone analysis.

From the results it was concluded that the puromycin resistance conferred by the fusion protein is comparable to the puromycin resistance conferred by the wild-type puromycin resistance gene. In conclusion, the inventive fusion shows the combined activity and function of both SEAP and pac containing fusion protein.

### 4. Example 4: Measurement of SEAP expression levels and Cell titer assay

### 4.1 SEAP Assay (Pierce Phosphatase Substrate Kit cat # 37620)

To 10µl of diluted cell-culture medium containing SEAP (diluted 1/10 in HBSS Gibco cat#14025-050) 100µ l of 1x Phosphatase Substrate Solution were added.
1 x Phosphatase Substrate Solution : (Pierce Phosphatase Substrate Kit cat # 37620)
4ml H₂O
1 ml 5x Concentrate Diethanolamine Substrate Buffer
1 PNPP Substrate Tablet

The solution was then incubated for 10-20 min at 37°C. The OD was read on a Spectrophotometer microplate reader at 405nm.

### 4.2 Cell Titer Assay (Promega CellTiter96 Aqueous One Cell Proliferation Assay cat # G3580)

To 50µl of cell suspension in 96-well plate 50µl of RPMI1640 (Gibco cat# 61870-010) 20µl of CellTiter 96Aqueous One Solution Reagent (Promega cat# G3580) were added. The solution was mixed well, incubated for 20-30 min at 37°C and the OD was read on a Spectrophotometer microplate reader at 490nm.

### 5. Example 5: SEAP HT Screening

Cells to be analyzed were transferred to a 96 well plate (5000-20000 cells per well) in ProCHO5/4.5 mM L-Glutamine/10 % Fetal Calf Serum and were incubated overnight at 37°C to allow them to attach to the bottom of the well.

On the next day the cells were washed 2x in ProCHO5/4.5 mM L-Glutamine and pulsed in 150 µl of the same medium for 24 h at 37°C after which the supernatant was harvested.

### 5.1 Measuring SEAP expression levels

10 µl of diluted supernatant (1/10 in HBSS) were added to 100 µl of phosphatase substrate solution (Pierce cat#37620) in a 96 well plate. The plate was incubated at 37°C for 10-15 minutes and OD was read at 490 nm.

### 5.2 Cell Titer Assay

After the pulse, the medium was replaced by a mix of 100 µl of RPMI1640 medium (Gibco cat#61870-010) plus 20 µl of CellTiter 96 Aqueous One Solution (Promega cat# G3580) and incubated at 37°C for 30 minutes. The OD was read at 490 nm.

The clones were ranked according the ratio of SEAP OD at 490 nm / CellTiter OD at 490 nm.

### 6. Example 6: Dual function of the inventive PuroLT fusion protein

CHO cells were transfected either with pmCMV(IE1)-SEAP-IRES-puro-279 or with pmCMV(IE1)-SEAP-IRES-PuroLT-280 as described in Example 3.1. Non-transfected cells were used as a control.

Upon selection with puromycin, pools of viable cells were obtained from the cells transfected either with pmCMV(IE1)-SEAP-IRES-puro-279 or with pmCMV(IE1)-SEAP-IRES-PuroLT-280 (see Figure 2, 5 and 6). To the contrary, no viable cells were obtained from the non-transfected cells. This demonstrated that puroLT conferred resistance to puromycin.

The cells transfected either with pmCMV(IE1)-SEAP-IRES-puro-279 or with pmCMV(IE1)-SEAP-IRES-PuroLT-280 were labeled with 4',5'-bis(1,3,2-dithioarsolan-2-yl)fluorescein as described in Example 2. The cells were either pre-incubated at 37°C or at 29°C before labeling.

The results are shown in Figure 8. No fluorescence was detected for cells transfected with pmCMV(IE1)-SEAP-IRES-puro-279, neither when the cells were pre-incubated at 29°C, nor when the cells were pre-incubated at 37°C. To the contrary, fluorescence was detected for cells transfected with pmCMV(IE1)-SEAP-IRES-puro-279 when the cells were preincubated at 29°C. This demonstrated that puroLT becomes fluorescent upon binding to 4',5'-bis(1,3,2-dithioarsolan-2-yl)fluorescein.

In conclusion, it was demonstrated that puroLT combines the functional properties of pac and of fluorescence upon binding with 4',5'-bis(1,3,2-dithioarsolan-2-yl)fluorescein. Accordingly, the inventive "puroLT" marker can be used both as a selectable marker in transfections due to its pac activity and as an easily detectable marker due to its fluorescence activity.

### 7. Example 7: Use of puroLT as a bifunctional marker for screening cells for high expression of a protein of interest

The dual function of the created fusion protein suggests that it should also have a dual impact. First, the inventive fusion protein should allow the isolation of stably transfected clones by their resistance to puromycin, and secondly, expression levels of said fusion should reflect expression levels of a physically connected gene of interest by measurement of fluorescence activity. In order to test this hypothesis, a series of clones from pools of cells stably transfected with inventive vectors were generated. Fluorescence activity and expression levels of the encoded proteins were measured.

CHO Cells were transfected with pmCMV(IE1)-SEAP-IRES-PuroLT-280 as described in Example 3.1. 176 clones were obtained. The clones were either screened using a classical high-throughput screening as described in Example 5, or labeled with 4',5'-bis(1,3,2-dithioarsolan-2-yl)fluorescein and visually selected for fluorescence intensity under a fluorescence microscope.

Eight clones expressing high levels of SEAP were selected using the classical high-throughput screening (referred to as "HT Screen"). Twelve highly fluorescent clones and ten moderately fluorescent clones were visually selected based on fluorescence intensity upon labeling with 4',5'-bis(1,3,2-dithioarsolan-2-yl)fluorescein (respectively referred to as "High LumioTag" and "Low LumioTag").

The High LumioTag and Low LumioTag clones were further tested for SEAP expression as described in Example 4.

The HT Screen clones were further labeled with 4',5'-bis(1,3,2-dithioarsolan-2-yl)fluorescein and examined under a fluorescence microscope.

SEAP expression levels and fluorescence intensity obtained for clones selected either using a classical high-throughput screening or for fluorescence intensity upon labeling with 4',5'-bis(1,3,2-dithioarsolan-2-yl)fluorescein were compared. The results are shown in Figure 3.

This experiment demonstrates that high SEAP expression level was always correlated with high fluorescence. For example, the High LumioTag clone No. 10 and the HT Screen clone No. 3 exhibit both higher fluorescence and higher SEAP expression level than the other clones.

This experiment further demonstrates that screening using the inventive fusion protein upon labeling with 4',5'-bis(1,3,2-dithioarsolan-2-yl)fluorescein allows to isolate clones that are as good SEAP expressors than those isolated using a standard high-throughput screening for SEAP expression levels

### Advantages

The present invention refers to a novel chimeric selection marker corresponding to a fusion protein comprising a peptide conferring resistance to an antibiotic, or a fragment, allelic variant, splice variant or mutein thereof, fused to at least one sequence comprising SEQ ID NO: 1, 2 or 3, wherein said fusion protein exhibits: (i) a resistance to said antibiotic; and (i) a fluorescence activity upon binding to a ligand of SEQ ID NO: 1, 2 or 3.

It has been demonstrated that the inventive fusion combines the functional properties of fluorescence measurement and of antibiotic selection (e.g. pac, see Example 2). Accordingly, the inventive marker can be used both as a selectable marker in stable transfections due to its antibiotic resistance and as an easily detectable marker due to its fluorescence activity.

Using the inventive fusion protein in HTS allows furthermore keeping at least the same chance for selecting high expressing clones, thereby allowing to reduce time and resources. In a classical HTS clone generation approach, the best clones are typically chosen on the basis of high titers for secreted proteins upon screening of more than 2,000 clones. Using the inventive fusion protein particularly leads to a reduction in sample size. This reduction may relate to the ease of use of the inventive approach and the associated reduction of sampling errors and assay variance related to ELISA high throughput screens. In addition, by selecting the 5 to 10 best clones per plate, the best clone per plate is expected to be selected. Thus, using the inventive fusion protein for screening 1,000 clones will reduce the number of clones to be analyzed to 50 to 100, and thus allow the avoidance of a second HTS.
In addition, it is important to note that the POI, expressed in correlation wit the inventive fusion protein, is not limited in its size, since fusion of a peptide, conferring resistance to an antibiotic, or a fragment, allelic variant, splice variant or mutein thereof, to a sequence comprising SEQ ID NO: 1, 2 or 3, leads to a small and thus efficient expression cassette. Furthermore, the two individual enzymes with so different activities and origins surprisingly retain their function in the inventive fusion protein as it is described here. The retained dual function clearly leads to a dual impact as the inventive fusion protein can truly be used to provide selectivity in stable transfection and acts as a chimeric selection marker for screening candidate clones for high expression of a gene of interest.

Summarizing the above, the usefulness of the inventive additional selection marker for the isolation of high-expressing clones for a protein of interest (POI), e.g. a therapeutic protein, has been demonstrated. It allows reducing time, cost and resources since (i) standardized product-independent and simple analysis is performed; and (ii) measuring fluorescence activity is an inexpensive assay. The present invention thus provides a powerful marker, which can both be used to provide selectivity in stable transfection and act as a detectable marker for screening candidate clones for high expression of a gene of interest.

### References:

•Altschul et al., (1990), J. Mol. Biol. 215, 403-410;
• Ausubel et al., (1987, 1992), Current Protocols in Molecular Biology, supra, Interscience, N.Y., 6.3 and 6.4;
•Blackwood and Kadonaga (1998), Science 281, 61-63;
•Berth et al. (2000); Biotechnol. Bioeng. 71, 266-273;
•Chesnut et al. (1996); J. Immunol. Methods 193, 17-27;
•Devereux et al., (1984), Nucleic Acids Res. 12, 387-395;
•Grantham, R. (1974), Science 185, 862-864;
•Griffin et al. (1998) Science, Jul 10; 281 (5374): 269-72);
•Kaufman et al. (1985), Mol. Cell Biol. 5, 1750-1759;
•Li, Harju and Peterson, (1999), Trends Genet. 15, 403-408;
•Messerle, Keil, and Koszinowski, (1991), J. Virol. 65, 1638-1643;
•Pearson (1990), Methods Enzymol. 183, 63-98;
•Pearson and Lipman (1988), Proc. Natl. Acad. Sci. U. S. A 85, 2444-2448;
•PCT/EP2004/050280;
•Sambrook, J. C., Fritsch, E. F., and Maniatis, T. (2001) Molecular Cloning: A Laboratory Manual, Cold Spring Harbor Laboratory Press, Cold Spring Harbor, NY);
•Seliger and McElroy, (1960), Arch. Biochem. Biophys. 88, 136-141);
•Smith and Waterman, (1981), J. Mol. Biol. 147, 195-197
•Stinchcomb et al., (1997) Nature, 282:39;
•US Patent 4,959,314 (Mark et al);
•US Patent 4,588,585 (Mark et al);
•US Patent 4,737,462 (Mark et al);
•US Patent 5,116,943 (Koths et al.);
•US Patent 4,965,195 (Namen et al.);
•US Patent 4,879,111 (Chong et al.);
•US Patent 5,017,691 (Lee et al.);
•US patent 4,904,584 (Shaw et al.);
•WO 87/03905;
•Wood, de Wet, Dewji, and DeLuca, (1984), Biochem Biophys. Res. Commun. 124, 592-596;

## Claims

1. A fusion protein comprising a peptide sequence conferring resistance to an antibiotic, or a fragment, allelic variant, splice variant or mutein thereof, fused to at least one sequence comprising SEQ ID NO: 1, 2 or 3, wherein said fusion protein exhibits:
(i) a resistance to said antibiotic, and
(ii) a fluorescence activity upon binding of a ligand to said sequence comprising SEQ ID NO: 1, 2 or 3.

2. The fusion protein of claim 1, wherein the fusion protein exhibits resistance to an antibiotic selected from any of the antibiotics neomycin, kanamycin, neomycin-kanamycin, hygromycin, gentamycin, chloramphenicol, puromycin, zeocin or bleomycin.

3. The fusion protein of claim 1, wherein said peptide, conferring resistance to an antibiotic, is selected from any of the sequences according to SEQ ID NOs:5, 7, 9, 11, 13, 15, 17, 19 or 21.

4. The fusion protein of claim 1, wherein said peptide, conferring resistance to an antibiotic, is encoded by any of the sequences according to SEQ ID NOs: 4, 6, 8, 10, 12, 14, 16, 18 or 20.

5. The fusion protein of claims 1 to 4, wherein said peptide, conferring resistance to an antibiotic, is a puromycin N-acetyl transferase (pac) derived from *Streptomyces alboniger.*

6. The fusion protein of claim 5, wherein said puromycin N-acetyl transferase (pac) comprises amino acids 2 to 199 of SEQ ID NO: 4.

7. The fusion protein of any of claims 1 to 6, wherein the 3' terminus of said SEQ ID NO: 1, 2 or 3, is fused to the 5' terminus of said peptide, conferring resistance to an antibiotic.

8. The fusion protein of any of claims 1 to 6, wherein the 3' terminus of said peptide, conferring resistance to an antibiotic, is fused to the 5' terminus of said SEQ ID NO: 1, 2 or 3.

9. The fusion protein of any of claims 1 to 6 and 8, wherein the fusion protein comprises the sequence according to SEQ ID NO: 23 or is encoded by the sequence according to SEQ ID NO: 22.

10. A nucleic acid encoding the fusion protein of any of claims 1 to 9.

11. A vector comprising the nucleic acid of claim 10.

12. The vector of claim 11, wherein said vector is an expression vector.

13. The vector of claim 12, wherein said vector further comprises a nucleic acid encoding a protein of interest.

14. The vector of claim 13, wherein the nucleic acid of claim 6 and the nucleic acid encoding a protein of interest (POI) are separated by an IRES sequence.

15. The vector of any of claims 11 to 14, wherein said vector comprises one promoter or promoter assembly, regulating the expression of both the fusion protein according to any of claims 1 to 9 and the expression of the protein of interest (POI).

16. The vector of any of claims 11 to 14, wherein said vector comprises at least two promoters, one regulating the expression of the fusion protein according to any of claims 1 to 9, and the other one regulating the expression of said protein of interest (POI).

17. The vector of any of claims 15 to 16, wherein said promoters are promoters of the murine CMV immediate early region.

18. The vector of any of claims 15 to 16, wherein said promoters are the IE1 and/or the IE2 promoters.

19. The vector of any of claims 11 to 18, wherein said vector further comprises an amplification marker, preferably selected from the group consisting of adenosine deaminase (ADA), dihydrofolate reductase (DHFR), multiple drug resistance gene (MDR), ornithine decarboxylase (ODC) and N-(phosphonacetyl)-L-aspartate resistance (CAD).

20. A cell comprising the nucleic acid of claim 10 or the vector according to any of claims 11 to 19.

21. The cell of claim 20, wherein said cell is selected from the group consisting of a non-human mammalian cell or a human cell.

22. A method of screening cells for expression of a protein of interest, said method comprising the steps of:
(i) transfecting cells with a vector according to any of claims 11 to 19;
(ii) selecting cell clones being resistant to an antibiotic as defined in claim 2;
(iii) incubating cells selected according to step (ii) with a solution containing a ligand with binding affinity to a sequence comprising SEQ ID NO: 1, 2 or 3 and fluorescent properties upon binding; and
(iv) detecting the fluorescence activity of cell clones selected according to step (ii) due to fluorescence of the ligand.

23. The method of claim 22, wherein the ligand is a fluorescein derivative.

24. The method of claim 23, wherein the ligand is a membrane permeable biarsenic fluorescein derivative,

25. The method of claim 24, wherein the ligand is selected from the group comprising 4',5'-bis(1,3,2-dithioarsolan-2-yl)fluorescein or a derivative thereof.

26. The method of any of claims 22 to 25, wherein said fluorescence activity is detected by FACS in step (iv).

27. The method of any of claims 22 to 26, wherein the fluorescence activity of at least 20, 50, 100, 500, 1.000, 5.000, 10.000, 50.000, 100.000, 500.000 or 1.000.000 cells is detected in step (iv).

28. The method of any of claims 22 to 27, further comprising the step of:
(v) selecting about 5% to about 20% of the cells assayed in step (iv), wherein the selected cells are those exhibiting highest fluorescence activity in step (iv).

29. The method of claim 28, further comprising the step of:
(vi) assaying the expression level of the protein of interest in the cells selected at the end of step (v).

30. A method of obtaining a cell line expressing a protein of interest, said method comprising the steps of:
(i) screening cells according to the method of any of claims 22 to 29;
(ii) selecting the cell(s) exhibiting the highest expression of said protein of interest; and
(iii) establishing a cell line from said cell.

31. A method of producing a protein of interest, said method comprising the steps of:
(i) culturing a cell line obtained according to the method of claim 30 under conditions which permit expression of said protein of interest; and
(ii) isolating said protein of interest.

32. The method of claim 31, further comprising the step of purifying said protein of interest.

33. The method of claim 32, further comprising the step of formulating said protein of interest into a pharmaceutical composition.

34. A method of producing the fusion protein according to any of claims 1 to 9, said method comprising the step of:
(i) culturing the cell according to any of claims 20 to 21 under conditions which permit expression of said fusion protein according; and
(ii) isolating said fusion protein.

35. The method of claim 34, further comprising the step of purifying said fusion protein according to any of claims 1 to 9.

36. Use of the fusion protein according to any of claims 1 to 9, of the nucleic acid according to claim 10, of the vector according to any of claims 11 to 19 or of the cell according to any of claims 20 or 21, for screening cells for expression of a protein of interest.


SEQUENCE LISTING

<110>  Applied Research Systems ARS Holding N.V.

<120>  System for screening screening cells for high expression of a
       protein of interest (POI)

<130>  SE02P002EP

<160>  25

<170>  PatentIn version 3.3

<210>  1
<211>  6
<212>  PRT
<213>  Artificial

<220>
<223>  Generic binding sequence of a ligand having fluorescent properties


<220>
<221>  misc_feature
<222>  (3)..(4)
<223>  Xaa = unknown or other

<400>  1

Cys Cys Xaa Xaa Cys Cys
1               5


<210>  2
<211>  6
<212>  PRT
<213>  Artificial

<220>
<223>  More specific binding sequence of a ligand having fluorescent
       properties


<220>
<221>  misc_feature
<222>  (3)..(4)
<223>  Xaa = Pro or Gly, preferably Pro at position 3 and Gly at
       position 4

<400>  2


Cys Cys Xaa Xaa Cys Cys
1               5


<210>  3
<211>  10
<212>  PRT
<213>  Artificial

<220>
<223>  Binding sequence of a ligand having fluorescent properties, N- and C-

terminally extended

<400> 3

Gly Cys Cys Pro Gly Cys Cys Gly Gly Gly
1               5                   10


<210> 4
<211> 600
<212> DNA
<213> Resistance gene for puromycin N-acetyltransferase derived from
Streptomyces alboniger


<220>
<221> CDS
<222> (1)..(600)

<400> 4
atg acc gag tac aag ccc acg gtg cgc ctc gcc acc cgc gac gac gtc      48
Met Thr Glu Tyr Lys Pro Thr Val Arg Leu Ala Thr Arg Asp Asp Val
1               5                   10                  15

ccc cgg gcc gta cgc acc ctc gcc gcc gcg ttc gcc gac tac ccc gcc      96
Pro Arg Ala Val Arg Thr Leu Ala Ala Ala Phe Ala Asp Tyr Pro Ala
            20                  25                  30

acg cgc cac acc gtc gac ccg gac cgc cac atc gag cgg gtc acc gag      144
Thr Arg His Thr Val Asp Pro Asp Arg His Ile Glu Arg Val Thr Glu
        35                  40                  45

ctg caa gaa ctc ttc ctc acg cgc gtc ggg ctc gac atc ggc aag gtg      192
Leu Gln Glu Leu Phe Leu Thr Arg Val Gly Leu Asp Ile Gly Lys Val
    50                  55                  60

tgg gtc gcg gac gac ggc gcc gcg gtg gcg gtc tgg acc acg ccg gag      240
Trp Val Ala Asp Asp Gly Ala Ala Val Ala Val Trp Thr Thr Pro Glu
65                  70                  75                  80

agc gtc gaa gcg ggg gcg gtg ttc gcc gag atc ggc ccg cgc atg gcc      288
Ser Val Glu Ala Gly Ala Val Phe Ala Glu Ile Gly Pro Arg Met Ala
                85                  90                  95

gag ttg agc ggt tcc cgg ctg gcc gcg cag caa cag atg gaa ggc ctc      336
Glu Leu Ser Gly Ser Arg Leu Ala Ala Gln Gln Gln Met Glu Gly Leu
            100                 105                 110

ctg gcg ccg cac cgg ccc aag gag ccc gcg tgg ttc ctg gcc acc gtc      384
Leu Ala Pro His Arg Pro Lys Glu Pro Ala Trp Phe Leu Ala Thr Val
        115                 120                 125

ggc gtc tcg ccc gac cac cag ggc aag ggt ctg ggc agc gcc gtc gtg      432
Gly Val Ser Pro Asp His Gln Gly Lys Gly Leu Gly Ser Ala Val Val
    130                 135                 140

ctc ccc gga gtg gag gcg gcc gag cgc gcc ggg gtg ccc gcc ttc ctg      480
Leu Pro Gly Val Glu Ala Ala Glu Arg Ala Gly Val Pro Ala Phe Leu
145                 150                 155                 160

gag acc tcc gcg ccc cgc aac ctc ccc ttc tac gag cgg ctc ggc ttc      528
Glu Thr Ser Ala Pro Arg Asn Leu Pro Phe Tyr Glu Arg Leu Gly Phe
                165                 170                 175

acc gtc acc gcc gac gtc gag gtg ccc gaa gga ccg cgc acc tgg tgc        576
Thr Val Thr Ala Asp Val Glu Val Pro Glu Gly Pro Arg Thr Trp Cys
            180                 185                 190

atg acc cgc aag ccc ggt gcc tga                                        600
Met Thr Arg Lys Pro Gly Ala
        195

<210>  5
<211>  199
<212>  PRT
<213>  Puromycin N-acetyltransferase derived from Streptomyces alboniger

<400>  5

Met Thr Glu Tyr Lys Pro Thr Val Arg Leu Ala Thr Arg Asp Asp Val
1               5                   10                  15

Pro Arg Ala Val Arg Thr Leu Ala Ala Ala Phe Ala Asp Tyr Pro Ala
            20                  25                  30

Thr Arg His Thr Val Asp Pro Asp Arg His Ile Glu Arg Val Thr Glu
        35                  40                  45

Leu Gln Glu Leu Phe Leu Thr Arg Val Gly Leu Asp Ile Gly Lys Val
    50                  55                  60

Trp Val Ala Asp Asp Gly Ala Ala Val Ala Val Trp Thr Thr Pro Glu
65                  70                  75                  80

Ser Val Glu Ala Gly Ala Val Phe Ala Glu Ile Gly Pro Arg Met Ala
                85                  90                  95

Glu Leu Ser Gly Ser Arg Leu Ala Ala Gln Gln Gln Met Glu Gly Leu
            100                 105                 110

Leu Ala Pro His Arg Pro Lys Glu Pro Ala Trp Phe Leu Ala Thr Val
        115                 120                 125

Gly Val Ser Pro Asp His Gln Gly Lys Gly Leu Gly Ser Ala Val Val
    130                 135                 140

Leu Pro Gly Val Glu Ala Ala Glu Arg Ala Gly Val Pro Ala Phe Leu
145                 150                 155                 160

Glu Thr Ser Ala Pro Arg Asn Leu Pro Phe Tyr Glu Arg Leu Gly Phe
                165                 170                 175

Thr Val Thr Ala Asp Val Glu Val Pro Glu Gly Pro Arg Thr Trp Cys

180                 185                 190

Met Thr Arg Lys Pro Gly Ala
        195

<210>  6
<211>  795
<212>  DNA
<213>  Artificial

<220>
<223>  Neomycin resistance gene (encoding Neomycin phosphotransferase
       type II)

<220>
<221>  CDS
<222>  (1)..(795)

<400>  6
atg gct aaa atg aga ata tca ccg gaa ttg aaa aaa ctg atc gaa aaa        48
Met Ala Lys Met Arg Ile Ser Pro Glu Leu Lys Lys Leu Ile Glu Lys
1               5                   10                  15

tac cgc tgc gta aaa gat acg gaa gga atg tct cct gct aag gta tat        96
Tyr Arg Cys Val Lys Asp Thr Glu Gly Met Ser Pro Ala Lys Val Tyr
            20                  25                  30

aag ctg gtg gga gaa aat gaa aac cta tat tta aaa atg acg gac agc       144
Lys Leu Val Gly Glu Asn Glu Asn Leu Tyr Leu Lys Met Thr Asp Ser
        35                  40                  45

cgg tat aaa ggg acc acc tat gat gtg gaa cgg gaa aag gac atg atg       192
Arg Tyr Lys Gly Thr Thr Tyr Asp Val Glu Arg Glu Lys Asp Met Met
    50                  55                  60

cta tgg ctg gaa gga aag ctg cct gtt cca aag gtc ctg cac ttt gaa       240
Leu Trp Leu Glu Gly Lys Leu Pro Val Pro Lys Val Leu His Phe Glu
65                  70                  75                  80

cgg cat gat ggc tgg agc aat ctg ctc atg agt gag gcc gat ggc gtc       288
Arg His Asp Gly Trp Ser Asn Leu Leu Met Ser Glu Ala Asp Gly Val
                85                  90                  95

ctt tgc tcg gaa gag tat gaa gat gaa caa agc cct gaa aag att atc       336
Leu Cys Ser Glu Glu Tyr Glu Asp Glu Gln Ser Pro Glu Lys Ile Ile
            100                 105                 110

gag ctg tat gcg gag tgc atc agg ctc ttt cac tcc atc gac ata tcg       384
Glu Leu Tyr Ala Glu Cys Ile Arg Leu Phe His Ser Ile Asp Ile Ser
        115                 120                 125

gat tgt ccc tat acg aat agc tta gac agc cgc tta gcc gaa ttg gat       432
Asp Cys Pro Tyr Thr Asn Ser Leu Asp Ser Arg Leu Ala Glu Leu Asp
    130                 135                 140

tac tta ctg aat aac gat ctg gcc gat gtg gat tgc gaa aac tgg gaa       480
Tyr Leu Leu Asn Asn Asp Leu Ala Asp Val Asp Cys Glu Asn Trp Glu
145                 150                 155                 160

gaa gac act cca ttt aaa gat ccg cgc gag ctg tat gat ttt tta aag       528

Glu Asp Thr Pro Phe Lys Asp Pro Arg Glu Leu Tyr Asp Phe Leu Lys
                165                 170                 175

acg gaa aag ccc gaa gag gaa ctt gtc ttt tcc cac ggc gac ctg gga        576
Thr Glu Lys Pro Glu Glu Glu Leu Val Phe Ser His Gly Asp Leu Gly
            180                 185                 190

gac agc aac atc ttt gtg aaa gat ggc aaa gta agt ggc ttt att gat        624
Asp Ser Asn Ile Phe Val Lys Asp Gly Lys Val Ser Gly Phe Ile Asp
        195                 200                 205

ctt ggg aga agc ggc agg gcg gac aag tgg tat gac att gcc ttc tgc        672
Leu Gly Arg Ser Gly Arg Ala Asp Lys Trp Tyr Asp Ile Ala Phe Cys
    210                 215                 220

gtc cgg tcg atc agg gag gat atc ggg gaa gaa cag tat gtc gag cta        720
Val Arg Ser Ile Arg Glu Asp Ile Gly Glu Glu Gln Tyr Val Glu Leu
225                 230                 235                 240

ttt ttt gac tta ctg ggg atc aag cct gat tgg gag aaa ata aaa tat        768
Phe Phe Asp Leu Leu Gly Ile Lys Pro Asp Trp Glu Lys Ile Lys Tyr
                245                 250                 255

tat att tta ctg gat gaa ttg ttt tag                                    795
Tyr Ile Leu Leu Asp Glu Leu Phe
            260


<210>  7
<211>  264
<212>  PRT
<213>  Artificial

<220>
<223>  Neomycin phosphotransferase type II

<400>  7

Met Ala Lys Met Arg Ile Ser Pro Glu Leu Lys Lys Leu Ile Glu Lys
1               5                   10                  15


Tyr Arg Cys Val Lys Asp Thr Glu Gly Met Ser Pro Ala Lys Val Tyr
            20                  25                  30


Lys Leu Val Gly Glu Asn Glu Asn Leu Tyr Leu Lys Met Thr Asp Ser
        35                  40                  45


Arg Tyr Lys Gly Thr Thr Tyr Asp Val Glu Arg Glu Lys Asp Met Met
    50                  55                  60


Leu Trp Leu Glu Gly Lys Leu Pro Val Pro Lys Val Leu His Phe Glu
65                  70                  75                  80


Arg His Asp Gly Trp Ser Asn Leu Leu Met Ser Glu Ala Asp Gly Val
                85                  90                  95


Leu Cys Ser Glu Glu Tyr Glu Asp Glu Gln Ser Pro Glu Lys Ile Ile

            100                 105                 110

Glu Leu Tyr Ala Glu Cys Ile Arg Leu Phe His Ser Ile Asp Ile Ser
        115                 120                 125

Asp Cys Pro Tyr Thr Asn Ser Leu Asp Ser Arg Leu Ala Glu Leu Asp
    130                 135                 140

Tyr Leu Leu Asn Asn Asp Leu Ala Asp Val Asp Cys Glu Asn Trp Glu
145                 150                 155                 160

Glu Asp Thr Pro Phe Lys Asp Pro Arg Glu Leu Tyr Asp Phe Leu Lys
                165                 170                 175

Thr Glu Lys Pro Glu Glu Glu Leu Val Phe Ser His Gly Asp Leu Gly
            180                 185                 190

Asp Ser Asn Ile Phe Val Lys Asp Gly Lys Val Ser Gly Phe Ile Asp
        195                 200                 205

Leu Gly Arg Ser Gly Arg Ala Asp Lys Trp Tyr Asp Ile Ala Phe Cys
    210                 215                 220

Val Arg Ser Ile Arg Glu Asp Ile Gly Glu Glu Gln Tyr Val Glu Leu
225                 230                 235                 240

Phe Phe Asp Leu Leu Gly Ile Lys Pro Asp Trp Glu Lys Ile Lys Tyr
                245                 250                 255

Tyr Ile Leu Leu Asp Glu Leu Phe
            260

<210> 8
<211> 795
<212> DNA
<213> Artificial

<220>
<223> Kanamycin resistance gene (encoding kanamycin phosphotransferase
type II)

<220>
<221> CDS
<222> (1)..(795)

<400> 8
atg att gaa caa gat gga ttg cac gca ggt tct ccg gcc gct tgg gtg 48
gag agg cta ttc ggc tat gac tgg gca caa cag aca atc ggc tgc tct 96
gat gcc gcc gtg ttc cgg ctg tca gcg cag ggg cgc ccg gtt ctt ttt 144
gtc aag acc gac ctg tcc ggt gcc ctg aat gaa ctg cag gac gag gca 192
gcg cgg cta tcg tgg ctg gcc acg acg ggc gtt cct tgc gca gct gtg 240
ctc gac gtt gtc act gaa gcg gga agg gac tgg ctg cta ttg ggc gaa 288
gtg ccg ggg cag gat ctc ctg tca tct cac ctt gct cct gcc gag aaa 336
gta tcc atc atg gct gat gca atg cgg cgg ctg cat acg ctt gat ccg 384
gct acc tgc cca ttc gac cac caa gcg aaa cat cgc atc gag cga gca 432
cgt act cgg atg gaa gcc ggt ctt gtc gat cag gat gat ctg gac gaa 480
gag cat cag ggg ctc gcg cca gcc gaa ctg ttc gcc agg ctc aag gcg 528
cgc atg ccc gac ggc gag gat ctc gtc gtg act cat ggc gat gcc tgc 576
ttg ccg aat atc atg gtg gaa aat ggc cgc ttt tct gga ttc atc gac 624
tgt ggc cgg ctg ggt gtg gcg gac cgc tat cag gac ata gcg ttg gct 672
acc cgt gat att gct gaa gag ctt ggc ggc gaa tgg gct gac cgc ttc 720
ctc gtg ctt tac ggt atc gcc gct ccc gat tcg cag cgc atc gcc ttc 768
tat cgc ctt ctt gac gag ttc ttc tga 795

<210>  9
<211>  264
<212>  PRT
<213>  Artificial

<220>
<223>  Kanamycin phosphotransferase type II

<400>  9

<210>  10
<211>  810
<212>  DNA
<213>  Artificial

<220>
<223>  Neomycin-kanamycin resistance gene (encoding neomycin-kanamycin
       phosphotransferase type II)

<220>
<221>  CDS
<222>  (10)..(804)

<400>  10
ctgcagacc atg att gag cag gat gga ctg cat gct ggc agc cct gct gct      51
tgg gtg gag aga ctg ttt ggc tac gat tgg gct cag cag acc att ggc        99
tgt tct gat gcc gct gtg ttc cgg ttg tct gca caa ggt cgg cct gtg       147
ttg ttt gtg aag aca gat ctg tct gga gcc ctc aac gaa ctc cag gat       195
gag gct gcc aga ctg agc tgg ttg gcc acc aca gga gtg cct tgt gct       243
gct gtg ctg gat gtg gtg act gag gct ggc aga gac tgg ctg ctg ctg       291
gga gag gtg cct ggc cag gac ctg ctg agc agc cac ctg gcc cca gct       339
gag aaa gtc agc atc atg gct gat gcc atg aga aga ctg cac acc ctg       387
gac cct gcc acc tgt cct ttc gac cac caa gcc aag cac aga att gag     435
aga gcc aga acc aga atg gag gct ggc ctg gtg gac cag gat gac ctc     483
gat gag gag cac cag ggc ctg gcc cca gcc gag ttg ttt gcc cgg ttg     531
aag gcc aga atg cca gat gga gag gat ttg gtg gtg aca cat gga gat     579
gcc tgt ctg cct aac atc atg gtg gag aat ggc aga ttc tct ggc ttc     627
att gac tgt ggc cgg ctg gga gtg gct gac aga tac cag gac att gca     675
ttg gcc acc aga gac att gca gag gag ttg gga gga gag tgg gct gac     723
aga ttc ctg gtg ctg tat ggc atc gct gcc cct gac agc cag aga atc     771
gcc ttc tac cgg ctg ctg gat gag ttc ttc tga gagctc                  810


<210>  11
<211>  264
<212>  PRT
<213>  Artificial

<220>
<223>  Neomycin-kanamycin phosphotransferase type II

<400>  11

<210>  12
<211>  1026
<212>  DNA
<213>  Artificial

<220>
<223>  Hygromycin resistance gene (encoding hygromycin resistance protein)

<220>

<221>  CDS
<222>  (1)..(1026)

<400>  12
atg aaa aag cct gaa ctc acc gcg acg tct gtc gag aag ttt ctg atc        48
Met Lys Lys Pro Glu Leu Thr Ala Thr Ser Val Glu Lys Phe Leu Ile
1               5                   10                  15

gaa aag ttc gac agc gtc tcc gac ctg atg cag ctc tcg gag ggc gaa        96
Glu Lys Phe Asp Ser Val Ser Asp Leu Met Gln Leu Ser Glu Gly Glu
            20                  25                  30

gaa tct cgt gct ttc agc ttc gat gta gga ggg cgt gga tat gtc ctg       144
Glu Ser Arg Ala Phe Ser Phe Asp Val Gly Gly Arg Gly Tyr Val Leu
        35                  40                  45

cgg gta aat agc tgc gcc gat ggt ttc tac aaa gat cgt tat gtt tat       192
Arg Val Asn Ser Cys Ala Asp Gly Phe Tyr Lys Asp Arg Tyr Val Tyr
    50                  55                  60

cgg cac ttt gca tcg gcc gcg ctc ccg att ccg gaa gtg ctt gac att       240
Arg His Phe Ala Ser Ala Ala Leu Pro Ile Pro Glu Val Leu Asp Ile
65                  70                  75                  80

ggg gaa ttc agc gag agc ctg acc tat tgc atc tcc cgc cgt gca cag       288
Gly Glu Phe Ser Glu Ser Leu Thr Tyr Cys Ile Ser Arg Arg Ala Gln
                85                  90                  95

ggt gtc acg ttg caa gac ctg cct gaa acc gaa ctg ccc gct gtt ctg       336
Gly Val Thr Leu Gln Asp Leu Pro Glu Thr Glu Leu Pro Ala Val Leu
            100                 105                 110

cag ccg gtc gcg gag gcc atg gat gcg atc gct gcg gcc gat ctt agc       384
Gln Pro Val Ala Glu Ala Met Asp Ala Ile Ala Ala Ala Asp Leu Ser
        115                 120                 125

cag acg agc ggg ttc ggc cca ttc gga ccg caa gga atc ggt caa tac       432
Gln Thr Ser Gly Phe Gly Pro Phe Gly Pro Gln Gly Ile Gly Gln Tyr
    130                 135                 140

act aca tgg cgt gat ttc ata tgc gcg att gct gat ccc cat gtg tat       480
Thr Thr Trp Arg Asp Phe Ile Cys Ala Ile Ala Asp Pro His Val Tyr
145                 150                 155                 160

cac tgg caa act gtg atg gac gac acc gtc agt gcg tcc gtc gcg cag       528
His Trp Gln Thr Val Met Asp Asp Thr Val Ser Ala Ser Val Ala Gln
                165                 170                 175

gct ctc gat gag ctg atg ctt tgg gcc gag gac tgc ccc gaa gtc cgg       576
Ala Leu Asp Glu Leu Met Leu Trp Ala Glu Asp Cys Pro Glu Val Arg
            180                 185                 190

cac ctc gtg cac gcg gat ttc ggc tcc aac aat gtc ctg acg gac aat       624
His Leu Val His Ala Asp Phe Gly Ser Asn Asn Val Leu Thr Asp Asn
        195                 200                 205

ggc cgc ata aca gcg gtc att gac tgg agc gag gcg atg ttc ggg gat       672
Gly Arg Ile Thr Ala Val Ile Asp Trp Ser Glu Ala Met Phe Gly Asp
    210                 215                 220

tcc caa tac gag gtc gcc aac atc ttc ttc tgg agg ccg tgg ttg gct       720
Ser Gln Tyr Glu Val Ala Asn Ile Phe Phe Trp Arg Pro Trp Leu Ala
225                 230                 235                 240

tgt atg gag cag cag acg cgc tac ttc gag cgg agg cat ccg gag ctt     768
Cys Met Glu Gln Gln Thr Arg Tyr Phe Glu Arg Arg His Pro Glu Leu
                245                 250                 255

gca gga tcg ccg cgg ctc cgg gcg tat atg ctc cgc att ggt ctt gac     816
Ala Gly Ser Pro Arg Leu Arg Ala Tyr Met Leu Arg Ile Gly Leu Asp
            260                 265                 270

caa ctc tat cag agc ttg gtt gac ggc aat ttc gat gat gca gct tgg     864
Gln Leu Tyr Gln Ser Leu Val Asp Gly Asn Phe Asp Asp Ala Ala Trp
        275                 280                 285

gcg cag ggt cga tgc gac gca atc gtc cga tcc gga gcc ggg act gtc     912
Ala Gln Gly Arg Cys Asp Ala Ile Val Arg Ser Gly Ala Gly Thr Val
    290                 295                 300

ggg cgt aca caa atc gcc cgc aga agc gcg gcc gtc tgg acc gat ggc     960
Gly Arg Thr Gln Ile Ala Arg Arg Ser Ala Ala Val Trp Thr Asp Gly
305                 310                 315                 320

tgt gta gaa gta ctc gcc gat agt gga aac cga cgc ccc agc act cgt     1008
Cys Val Glu Val Leu Ala Asp Ser Gly Asn Arg Arg Pro Ser Thr Arg
                325                 330                 335

ccg agg gca aag gaa tag                                             1026
Pro Arg Ala Lys Glu
            340


<210>  13
<211>  341
<212>  PRT
<213>  Artificial

<220>
<223>  Hygromycin resistance protein

<400>  13

Met Lys Lys Pro Glu Leu Thr Ala Thr Ser Val Glu Lys Phe Leu Ile
1               5                   10                  15


Glu Lys Phe Asp Ser Val Ser Asp Leu Met Gln Leu Ser Glu Gly Glu
            20                  25                  30


Glu Ser Arg Ala Phe Ser Phe Asp Val Gly Gly Arg Gly Tyr Val Leu
        35                  40                  45


Arg Val Asn Ser Cys Ala Asp Gly Phe Tyr Lys Asp Arg Tyr Val Tyr
    50                  55                  60


Arg His Phe Ala Ser Ala Ala Leu Pro Ile Pro Glu Val Leu Asp Ile
65                  70                  75                  80


Gly Glu Phe Ser Glu Ser Leu Thr Tyr Cys Ile Ser Arg Arg Ala Gln
                85                  90                  95

Gly Val Thr Leu Gln Asp Leu Pro Glu Thr Glu Leu Pro Ala Val Leu
            100                 105                 110

Gln Pro Val Ala Glu Ala Met Asp Ala Ile Ala Ala Ala Asp Leu Ser
        115                 120                 125

Gln Thr Ser Gly Phe Gly Pro Phe Gly Pro Gln Gly Ile Gly Gln Tyr
    130                 135                 140

Thr Thr Trp Arg Asp Phe Ile Cys Ala Ile Ala Asp Pro His Val Tyr
145                 150                 155                 160

His Trp Gln Thr Val Met Asp Asp Thr Val Ser Ala Ser Val Ala Gln
                165                 170                 175

Ala Leu Asp Glu Leu Met Leu Trp Ala Glu Asp Cys Pro Glu Val Arg
            180                 185                 190

His Leu Val His Ala Asp Phe Gly Ser Asn Asn Val Leu Thr Asp Asn
        195                 200                 205

Gly Arg Ile Thr Ala Val Ile Asp Trp Ser Glu Ala Met Phe Gly Asp
    210                 215                 220

Ser Gln Tyr Glu Val Ala Asn Ile Phe Phe Trp Arg Pro Trp Leu Ala
225                 230                 235                 240

Cys Met Glu Gln Gln Thr Arg Tyr Phe Glu Arg Arg His Pro Glu Leu
                245                 250                 255

Ala Gly Ser Pro Arg Leu Arg Ala Tyr Met Leu Arg Ile Gly Leu Asp
            260                 265                 270

Gln Leu Tyr Gln Ser Leu Val Asp Gly Asn Phe Asp Asp Ala Ala Trp
        275                 280                 285

Ala Gln Gly Arg Cys Asp Ala Ile Val Arg Ser Gly Ala Gly Thr Val
    290                 295                 300

Gly Arg Thr Gln Ile Ala Arg Arg Ser Ala Ala Val Trp Thr Asp Gly
305                 310                 315                 320

Cys Val Glu Val Leu Ala Asp Ser Gly Asn Arg Arg Pro Ser Thr Arg
                325                 330                 335

Pro Arg Ala Lys Glu
            340

<210>  14
<211>  534
<212>  DNA
<213>  Artificial

<220>
<223>  Gentamycin resistance gene (encoding gentamycin acetyl
       transferase)


<220>
<221>  CDS
<222>  (1)..(534)

<400>  14
atg tta cgc agc agc aac gat gtt acg cag cag ggc agt cgc cct aaa        48
Met Leu Arg Ser Ser Asn Asp Val Thr Gln Gln Gly Ser Arg Pro Lys
1               5                   10                  15

aca aag tta ggt ggc tca agt atg ggc atc att cgc aca tgt agg ctc        96
Thr Lys Leu Gly Gly Ser Ser Met Gly Ile Ile Arg Thr Cys Arg Leu
            20                  25                  30

ggc cct gac caa gtc aaa tcc atg cgg gct gct ctt gat ctt ttc ggt       144
Gly Pro Asp Gln Val Lys Ser Met Arg Ala Ala Leu Asp Leu Phe Gly
        35                  40                  45

cgt gag ttc gga gac gta gcc acc tac tcc caa cat cag ccg gac tcc       192
Arg Glu Phe Gly Asp Val Ala Thr Tyr Ser Gln His Gln Pro Asp Ser
    50                  55                  60

gat tac ctc ggg aac ttg ctc cgt agt aag aca ttc atc gcg ctt gct       240
Asp Tyr Leu Gly Asn Leu Leu Arg Ser Lys Thr Phe Ile Ala Leu Ala
65                  70                  75                  80

gcc ttc gac caa gaa gcg gtt gtt ggc gct ctc gcg gct tac gtt ctg       288
Ala Phe Asp Gln Glu Ala Val Val Gly Ala Leu Ala Ala Tyr Val Leu
                85                  90                  95

ccc agg ttt gag cag ccg cgt agt gag atc tat atc tat gat ctc gca       336
Pro Arg Phe Glu Gln Pro Arg Ser Glu Ile Tyr Ile Tyr Asp Leu Ala
            100                 105                 110

gtc tcc ggc gag cac cgg agg cag ggc att gcc acc gcg ctc atc aat       384
Val Ser Gly Glu His Arg Arg Gln Gly Ile Ala Thr Ala Leu Ile Asn
        115                 120                 125

ctc ctc aag cat gag gcc aac gcg ctt ggt gct tat gtg atc tac gtg       432
Leu Leu Lys His Glu Ala Asn Ala Leu Gly Ala Tyr Val Ile Tyr Val
    130                 135                 140

caa gca gat tac ggt gac gat ccc gca gtg gct ctc tat aca aag ttg       480
Gln Ala Asp Tyr Gly Asp Asp Pro Ala Val Ala Leu Tyr Thr Lys Leu
145                 150                 155                 160

ggc ata cgg gaa gaa gtg atg cac ttt gat atc gac cca agt acc gcc       528
Gly Ile Arg Glu Glu Val Met His Phe Asp Ile Asp Pro Ser Thr Ala
                165                 170                 175

acc taa                                                               534
Thr

<210>  15
<211>  177
<212>  PRT
<213>  Artificial

<220>
<223>  Gentamycin acetyl transferase

<400>  15

Met Leu Arg Ser Ser Asn Asp Val Thr Gln Gln Gly Ser Arg Pro Lys
1               5                   10                  15

Thr Lys Leu Gly Gly Ser Ser Met Gly Ile Ile Arg Thr Cys Arg Leu
            20                  25                  30

Gly Pro Asp Gln Val Lys Ser Met Arg Ala Ala Leu Asp Leu Phe Gly
        35                  40                  45

Arg Glu Phe Gly Asp Val Ala Thr Tyr Ser Gln His Gln Pro Asp Ser
    50                  55                  60

Asp Tyr Leu Gly Asn Leu Leu Arg Ser Lys Thr Phe Ile Ala Leu Ala
65                  70                  75                  80

Ala Phe Asp Gln Glu Ala Val Val Gly Ala Leu Ala Ala Tyr Val Leu
                85                  90                  95

Pro Arg Phe Glu Gln Pro Arg Ser Glu Ile Tyr Ile Tyr Asp Leu Ala
            100                 105                 110

Val Ser Gly Glu His Arg Arg Gln Gly Ile Ala Thr Ala Leu Ile Asn
        115                 120                 125

Leu Leu Lys His Glu Ala Asn Ala Leu Gly Ala Tyr Val Ile Tyr Val
    130                 135                 140

Gln Ala Asp Tyr Gly Asp Asp Pro Ala Val Ala Leu Tyr Thr Lys Leu
145                 150                 155                 160

Gly Ile Arg Glu Glu Val Met His Phe Asp Ile Asp Pro Ser Thr Ala
                165                 170                 175

Thr

<210>  16
<211>  459

<212>  DNA
<213>  Artificial

<220>
<223>  Chloramphenicol resistance gene (encoding chloramphenicol
       acetyltransferase)


<220>
<221>  CDS
<222>  (1)..(459)

<400>  16
atg aac ttt aat aaa att gat tta gac aat tgg aag aga aaa gag ata       48
Met Asn Phe Asn Lys Ile Asp Leu Asp Asn Trp Lys Arg Lys Glu Ile
1               5                   10                  15

ttt aat cat tat ttg aac caa caa acg act ttt agt ata acc aca gaa       96
Phe Asn His Tyr Leu Asn Gln Gln Thr Thr Phe Ser Ile Thr Thr Glu
            20                  25                  30

att gat att agt gtt tta tac cga aac ata aaa caa gaa gga tat aaa      144
Ile Asp Ile Ser Val Leu Tyr Arg Asn Ile Lys Gln Glu Gly Tyr Lys
        35                  40                  45

ttt tac cct gca ttt att ttc tta gtg aca agg gtg ata aac tca aat      192
Phe Tyr Pro Ala Phe Ile Phe Leu Val Thr Arg Val Ile Asn Ser Asn
    50                  55                  60

aca gct ttt aga act ggt tac aat agc gac gga gag tta ggt tat tgg      240
Thr Ala Phe Arg Thr Gly Tyr Asn Ser Asp Gly Glu Leu Gly Tyr Trp
65                  70                  75                  80

gat aag tta gag cca ctt tat aca att ttt gat ggt gta tct aaa aca      288
Asp Lys Leu Glu Pro Leu Tyr Thr Ile Phe Asp Gly Val Ser Lys Thr
                85                  90                  95

ttc tct ggt att tgg act cct gta aag aat gac ttc aaa gag ttt tat      336
Phe Ser Gly Ile Trp Thr Pro Val Lys Asn Asp Phe Lys Glu Phe Tyr
            100                 105                 110


gat tta tac ctt tct gat gta gag aaa tat aat ggt tcg ggg aaa ttg      384
Asp Leu Tyr Leu Ser Asp Val Glu Lys Tyr Asn Gly Ser Gly Lys Leu
        115                 120                 125

ttt ccc aaa aca cct ata cct gaa aaa tgc ttt ttc tct ttc tat tat      432
Phe Pro Lys Thr Pro Ile Pro Glu Lys Cys Phe Phe Ser Phe Tyr Tyr
    130                 135                 140

tcc atg gac ttc att tac tgg gtt taa                                  459
Ser Met Asp Phe Ile Tyr Trp Val
145                 150


<210>  17
<211>  152
<212>  PRT
<213>  Artificial

<220>
<223>  Chloramphenicol acetyltransferase

<400>  17

Met Asn Phe Asn Lys Ile Asp Leu Asp Asn Trp Lys Arg Lys Glu Ile
1               5                   10                  15

Phe Asn His Tyr Leu Asn Gln Gln Thr Thr Phe Ser Ile Thr Thr Glu
            20                  25                  30

Ile Asp Ile Ser Val Leu Tyr Arg Asn Ile Lys Gln Glu Gly Tyr Lys
        35                  40                  45

Phe Tyr Pro Ala Phe Ile Phe Leu Val Thr Arg Val Ile Asn Ser Asn
    50                  55                  60

Thr Ala Phe Arg Thr Gly Tyr Asn Ser Asp Gly Glu Leu Gly Tyr Trp
65                  70                  75                  80

Asp Lys Leu Glu Pro Leu Tyr Thr Ile Phe Asp Gly Val Ser Lys Thr
                85                  90                  95

Phe Ser Gly Ile Trp Thr Pro Val Lys Asn Asp Phe Lys Glu Phe Tyr
            100                 105                 110

Asp Leu Tyr Leu Ser Asp Val Glu Lys Tyr Asn Gly Ser Gly Lys Leu
        115                 120                 125

Phe Pro Lys Thr Pro Ile Pro Glu Lys Cys Phe Phe Ser Phe Tyr Tyr
    130                 135                 140

Ser Met Asp Phe Ile Tyr Trp Val
145                 150


<210>  18
<211>  375
<212>  DNA
<213>  Artificial

<220>
<223>  Zeomycin resistance gene


<220>
<221>  CDS
<222>  (1)..(375)

<400>  18
atg gcc aag ttg acc agt gcc gtt ccg gtg ctc acc gcg cgc gac gtc        48
Met Ala Lys Leu Thr Ser Ala Val Pro Val Leu Thr Ala Arg Asp Val
1               5                   10                  15

gcc gga gcg gtc gag ttc tgg acc gac cgg ctc ggg ttc tcc cgg gac        96
Ala Gly Ala Val Glu Phe Trp Thr Asp Arg Leu Gly Phe Ser Arg Asp
            20                  25                  30

ttc gtg gag gac gac ttc gcc ggt gtg gtc cgg gac gac gtg acc ctg          144
Phe Val Glu Asp Asp Phe Ala Gly Val Val Arg Asp Asp Val Thr Leu
        35                  40                  45

ttc atc agc gcg gtc cag gac cag gtg gtg ccg gac aac acc ctg gcc          192
Phe Ile Ser Ala Val Gln Asp Gln Val Val Pro Asp Asn Thr Leu Ala
    50                  55                  60

tgg gtg tgg gtg cgc ggc ctg gac gag ctg tac gcc gag tgg tcg gag          240
Trp Val Trp Val Arg Gly Leu Asp Glu Leu Tyr Ala Glu Trp Ser Glu
65                  70                  75                  80

gtc gtg tcc acg aac ttc cgg gac gcc tcc ggg ccg gcc atg acc gag          288
Val Val Ser Thr Asn Phe Arg Asp Ala Ser Gly Pro Ala Met Thr Glu
                85                  90                  95

atc ggc gag cag ccg tgg ggg cgg gag ttc gcc ctg cgc gac ccg gcc          336
Ile Gly Glu Gln Pro Trp Gly Arg Glu Phe Ala Leu Arg Asp Pro Ala
            100                 105                 110

ggc aac tgc gtg cac ttc gtg gcc gag gag cag gac tga                      375
Gly Asn Cys Val His Phe Val Ala Glu Glu Gln Asp
        115                 120


<210>  19
<211>  124
<212>  PRT
<213>  Artificial

<220>
<223>  Zeomycin resistance protein

<400>  19

Met Ala Lys Leu Thr Ser Ala Val Pro Val Leu Thr Ala Arg Asp Val
1               5                   10                  15


Ala Gly Ala Val Glu Phe Trp Thr Asp Arg Leu Gly Phe Ser Arg Asp
            20                  25                  30


Phe Val Glu Asp Asp Phe Ala Gly Val Val Arg Asp Asp Val Thr Leu
        35                  40                  45


Phe Ile Ser Ala Val Gln Asp Gln Val Val Pro Asp Asn Thr Leu Ala
    50                  55                  60


Trp Val Trp Val Arg Gly Leu Asp Glu Leu Tyr Ala Glu Trp Ser Glu
65                  70                  75                  80


Val Val Ser Thr Asn Phe Arg Asp Ala Ser Gly Pro Ala Met Thr Glu
                85                  90                  95


Ile Gly Glu Gln Pro Trp Gly Arg Glu Phe Ala Leu Arg Asp Pro Ala
            100                 105                 110

Gly Asn Cys Val His Phe Val Ala Glu Glu Gln Asp
        115                 120

<210>  20
<211>  435
<212>  DNA
<213>  Artificial

<220>
<223>  bleomycin resistance gene

<220>
<221>  CDS
<222>  (1)..(435)

<400>  20
atg aaa cca aga att agc atg att act ctc ggg gtt aaa gac ctt gaa        48
Met Lys Pro Arg Ile Ser Met Ile Thr Leu Gly Val Lys Asp Leu Glu
1               5                   10                  15

aag tca gtc gta ttt tat cgt gat ggc tta gga ttt cct caa aaa gaa        96
Lys Ser Val Val Phe Tyr Arg Asp Gly Leu Gly Phe Pro Gln Lys Glu
            20                  25                  30

tcg cca cca tca gtc gcc ttt ttt act ctg aat ggt aca tgg tta ggt       144
Ser Pro Pro Ser Val Ala Phe Phe Thr Leu Asn Gly Thr Trp Leu Gly
        35                  40                  45

ttg tat gat cgt gat gcc tta gca gaa gat gct caa gtg gct cct agt       192
Leu Tyr Asp Arg Asp Ala Leu Ala Glu Asp Ala Gln Val Ala Pro Ser
    50                  55                  60

gat aat act gag caa tct ttt tct ggt ttt gca ctt gcg cat aat gtg       240
Asp Asn Thr Glu Gln Ser Phe Ser Gly Phe Ala Leu Ala His Asn Val
65                  70                  75                  80

aag tct gaa acg gaa gtc gat caa gtg ttg act gaa gta gaa gcc gcg       288
Lys Ser Glu Thr Glu Val Asp Gln Val Leu Thr Glu Val Glu Ala Ala
                85                  90                  95

ggc gcg act gtt acc aag cgc ggt cag aaa gtg ttt tgg ggt ggg tat       336
Gly Ala Thr Val Thr Lys Arg Gly Gln Lys Val Phe Trp Gly Gly Tyr
            100                 105                 110

tcc ggt tac ttc aaa gat ctt gat ggc tat tta tgg gag gtt gcc tat       384
Ser Gly Tyr Phe Lys Asp Leu Asp Gly Tyr Leu Trp Glu Val Ala Tyr
        115                 120                 125

aac ccc ttt tgt tgg ata ggc cct gaa gat tcg att gat ata aga tgg       432
Asn Pro Phe Cys Trp Ile Gly Pro Glu Asp Ser Ile Asp Ile Arg Trp
    130                 135                 140

tga                                                                   435

<210>  21
<211>  144
<212>  PRT
<213>  Artificial

<220>
<223>  bleomycin resistance protein

<400>  21

Met Lys Pro Arg Ile Ser Met Ile Thr Leu Gly Val Lys Asp Leu Glu
1               5                   10                  15

Lys Ser Val Val Phe Tyr Arg Asp Gly Leu Gly Phe Pro Gln Lys Glu
            20                  25                  30

Ser Pro Pro Ser Val Ala Phe Phe Thr Leu Asn Gly Thr Trp Leu Gly
        35                  40                  45

Leu Tyr Asp Arg Asp Ala Leu Ala Glu Asp Ala Gln Val Ala Pro Ser
    50                  55                  60

Asp Asn Thr Glu Gln Ser Phe Ser Gly Phe Ala Leu Ala His Asn Val
65                  70                  75                  80

Lys Ser Glu Thr Glu Val Asp Gln Val Leu Thr Glu Val Glu Ala Ala
                85                  90                  95

Gly Ala Thr Val Thr Lys Arg Gly Gln Lys Val Phe Trp Gly Gly Tyr
            100                 105                 110

Ser Gly Tyr Phe Lys Asp Leu Asp Gly Tyr Leu Trp Glu Val Ala Tyr
        115                 120                 125

Asn Pro Phe Cys Trp Ile Gly Pro Glu Asp Ser Ile Asp Ile Arg Trp
    130                 135                 140


<210>  22
<211>  630
<212>  DNA
<213>  Artificial

<220>
<223>  sequence encoding an exemplary inventive fusion protein,
       comprising as a first component a puromycin N-acetyltransferase
       sequence and as a second component a sequence according to SEQ ID
       NO: 1 to 3


<220>
<221>  CDS
<222>  (1)..(630)

<400>  22
atg acc gag tac aag ccc acg gtg cgc ctc gcc acc cgc gac gac gtc       48
Met Thr Glu Tyr Lys Pro Thr Val Arg Leu Ala Thr Arg Asp Asp Val
1               5                   10                  15

ccc cgg gcc gta cgc acc ctc gcc gcc gcg ttc gcc gac tac ccc gcc       96

Pro Arg Ala Val Arg Thr Leu Ala Ala Ala Phe Ala Asp Tyr Pro Ala
            20                  25                  30

acg cgc cac acc gtc gac ccg gac cgc cac atc gag cgg gtc acc gag      144
Thr Arg His Thr Val Asp Pro Asp Arg His Ile Glu Arg Val Thr Glu
        35                  40                  45

ctg caa gaa ctc ttc ctc acg cgc gtc ggg ctc gac atc ggc aag gtg      192
Leu Gln Glu Leu Phe Leu Thr Arg Val Gly Leu Asp Ile Gly Lys Val
    50                  55                  60

tgg gtc gcg gac gac ggc gcc gcg gtg gcg gtc tgg acc acg ccg gag      240
Trp Val Ala Asp Asp Gly Ala Ala Val Ala Val Trp Thr Thr Pro Glu
65                  70                  75                  80

agc gtc gaa gcg ggg gcg gtg ttc gcc gag atc ggc ccg cgc atg gcc      288
Ser Val Glu Ala Gly Ala Val Phe Ala Glu Ile Gly Pro Arg Met Ala
                85                  90                  95

gag ttg agc ggt tcc cgg ctg gcc gcg cag caa cag atg gaa ggc ctc      336
Glu Leu Ser Gly Ser Arg Leu Ala Ala Gln Gln Gln Met Glu Gly Leu
            100                 105                 110

ctg gcg ccg cac cgg ccc aag gag ccc gcg tgg ttc ctg gcc acc gtc      384
Leu Ala Pro His Arg Pro Lys Glu Pro Ala Trp Phe Leu Ala Thr Val
        115                 120                 125

ggc gtc tcg ccc gac cac cag ggc aag ggt ctg ggc agc gcc gtc gtg      432
Gly Val Ser Pro Asp His Gln Gly Lys Gly Leu Gly Ser Ala Val Val
    130                 135                 140

ctc ccc gga gtg gag gcg gcc gag cgc gcc ggg gtg ccc gcc ttc ctg      480
Leu Pro Gly Val Glu Ala Ala Glu Arg Ala Gly Val Pro Ala Phe Leu
145                 150                 155                 160

gag acc tcc gcg ccc cgc aac ctc ccc ttc tac gag cgg ctc ggc ttc      528
Glu Thr Ser Ala Pro Arg Asn Leu Pro Phe Tyr Glu Arg Leu Gly Phe
                165                 170                 175

acc gtc acc gcc gac gtc gag gtg ccc gaa gga ccg cgc acc tgg tgc      576
Thr Val Thr Ala Asp Val Glu Val Pro Glu Gly Pro Arg Thr Trp Cys
            180                 185                 190

atg acc cgc aag ccc ggt gcc ggc tgt tgt cct ggc tgt tgc ggt ggc      624
Met Thr Arg Lys Pro Gly Ala Gly Cys Cys Pro Gly Cys Cys Gly Gly
        195                 200                 205

ggc tag                                                              630
Gly

<210>  23
<211>  209
<212>  PRT
<213>  Artificial

<220>
<223>  Synthetic Construct

<400>  23

Met Thr Glu Tyr Lys Pro Thr Val Arg Leu Ala Thr Arg Asp Asp Val

1               5                   10                  15

Pro Arg Ala Val Arg Thr Leu Ala Ala Ala Phe Ala Asp Tyr Pro Ala
            20                  25                  30

Thr Arg His Thr Val Asp Pro Asp Arg His Ile Glu Arg Val Thr Glu
        35                  40                  45

Leu Gln Glu Leu Phe Leu Thr Arg Val Gly Leu Asp Ile Gly Lys Val
    50                  55                  60

Trp Val Ala Asp Asp Gly Ala Ala Val Ala Val Trp Thr Thr Pro Glu
65                  70                  75                  80

Ser Val Glu Ala Gly Ala Val Phe Ala Glu Ile Gly Pro Arg Met Ala
                85                  90                  95

Glu Leu Ser Gly Ser Arg Leu Ala Ala Gln Gln Gln Met Glu Gly Leu
            100                 105                 110

Leu Ala Pro His Arg Pro Lys Glu Pro Ala Trp Phe Leu Ala Thr Val
        115                 120                 125

Gly Val Ser Pro Asp His Gln Gly Lys Gly Leu Gly Ser Ala Val Val
    130                 135                 140

Leu Pro Gly Val Glu Ala Ala Glu Arg Ala Gly Val Pro Ala Phe Leu
145                 150                 155                 160

Glu Thr Ser Ala Pro Arg Asn Leu Pro Phe Tyr Glu Arg Leu Gly Phe
                165                 170                 175

Thr Val Thr Ala Asp Val Glu Val Pro Glu Gly Pro Arg Thr Trp Cys
            180                 185                 190

Met Thr Arg Lys Pro Gly Ala Gly Cys Cys Pro Gly Cys Cys Gly Gly
        195                 200                 205

Gly

<210>  24
<211>  22
<212>  DNA
<213>  Artificial

<220>
<223>  Primer oSerono1206

<400> 24
gtggctgctt atggtgacaa tc 22

<210> 25
<211> 67
<212> DNA
<213> Artificial

<220>
<223> Primer oSerono1239

<400> 25
cgcgctagct cattactagc cgccaccgca acagccagga caacagccgg caccgggctt 60

gcgggtc 67